# FASCICULE DE BREVET EUROPEEN

(11) **EP 0 627 483 B1**
(45) Date de publication et mention de la délivrance du brevet: **23.12.1998**
(21) Numéro de dépôt: 94401159.2
(22) Date de dépôt: 25.05.1994
(51) Int. Cl.: C10L 1/14, C10L 10/00, C08G 65/26, C07D 498/04, C10L 1/22

(54) **Formulation d'additifs pour carburants comprenant au moins un composé imidazo-oxazole alkoxylé**
Mindestens eine, alkoxylierte Imidazo-Oxazol Verbindung enthaltende Treibstoffzusätzeformulierung
Additives formulated for motor-fuels containing at least one alkoxylated imidazo-oxazole compound

(30) Priorité: 02.06.1993 FR 9306688
(43) Date de publication de la demande: 07.12.1994
(73) Titulaire: INSTITUT FRANCAIS DU PETROLE, 92502 Rueil-Malmaison (FR); ELF ANTAR FRANCE, 92078 Paris La Defense (FR)
(72) Inventeur: Delhomme, Henri, F-69110 Saintes Foy Les Lyons (FR); Gaillard, Jean, F-38400 Saint Martin d'Heres (FR); Mulard, Philippe, F-69780 Saint Pierre de Chandieu (FR); Eber, Danièle, F-69005 Lyon (FR)
(74) Mandataire: Andreeff, François

(56) Documents cités:
- EP-A- 0 180 910
- EP-A- 0 444 770
- EP-A- 0 530 094
- CH-A- 494 770
- US-A- 2 987 515
- US-A- 3 251 664
- US-A- 3 560 520
- PATENT ABSTRACTS OF JAPAN vol. 006, no. 177 (C-124) & JP-A-57 094 069 (CANON INC.)
- CHEMICAL ABSTRACTS, vol. 93, no. 26, 29 Décembre 1980, Columbus, Ohio, US; abstract no. 240687t, G. SZATLMAYER
- CHEMICAL ABSTRACTS, vol. 77, no. 4, 24 Juillet 1972, Columbus, Ohio, US; abstract no. 20861z,
- POLYMER BULLETIN, vol.6, no.5-6, 1982, HEIDELBERG DE pages 344 - 349 Y.SANO ET AL.

## Description

La présente invention concerne des formulations d'additifs, notamment pour carburants, comprenant au moins un composé imidazo-oxazole alkoxylé, au moins un composé choisi dans le groupe formé par les détergent-dispersants et au moins un produit choisi dans le groupe formé par les huiles lubrifiantes minérales ou synthétiques et les polyglycols solubles dans ledit carburant. Ces formulations sont utilisables comme additifs multifonctionnels pour les carburants employés dans les moteurs à combustion interne et en particulier pour ceux employés dans les moteurs à allumage commandé.

L'utilisation de carburants conventionnels conduit très souvent à l'encrassement des différentes parties du moteur par suite de la vaporisation et de la combustion incomplètes du carburant dans le système d'admission et dans les chambres de combustion.

En particulier, dans le cas des moteurs à allumage commandé, la formation et l'accumulation de dépôts dans les chambres de combustion perturbent les conditions de fonctionnement normales du moteur.

Ces dépôts modifient significativement les échanges thermiques entre les chambres de combustion et le système de refroidissement du moteur en formant une couche à caractère isolant.

Il s'ensuit une augmentation de la température dans les chambres à laquelle le mélange gazeux admis est soumis. L'auto-inflammation de ces gaz est alors favorisée, ce qui provoque l'apparition du phénomène bien connu de cliquetis du moteur.

Par ailleurs, l'accumulation de ces dépôts dans les chambres de combustion peut aboutir à une réduction du volume de la zone de combustion qui se traduit alors par une augmentation du taux de compression du moteur. Ce phénomène favorise également l'apparition du cliquetis. Par ailleurs les dépôts qui se forment dans les diverses parties du moteur en contact avec le carburant peuvent absorber partiellement une partie de ce carburant contribuant ainsi à une modification du mélange comburant-combustible avec une phase d'appauvrissement en combustible lors de l'absorption et une phase d'enrichissement dans le cas d'une désorption de ce carburant. La modification de la richesse du mélange carburant-air ne permet plus au moteur de travailler dans des conditions optimales.

Afin de remédier à l'encrassement il est possible de procéder à un nettoyage périodique, particulièrement onéreux, des organes concernés en particulier des soupapes.

L'accumulation de dépôts dans les moteurs et en particulier sur les soupapes d'admission peut également être réduite par l'utilisation de carburants contenant certains additifs, par exemple des additifs du type détergent éventuellement combinés par exemple avec des additifs anticorrosion ou antidépôts pour chambre de combustion.

Les additifs, bien connus dans le commerce, par exemple ceux du type polyisobutène-amine, sont habituellement associés à une huile minérale ou synthétique et sont susceptibles de provoquer un encrassement accru des chambres de combustion et donc une augmentation de l'exigence en octane du moteur avec une plus grande sensibilité au phénomène de cliquetis.

Parmi les nombreux additifs décrits dans l'art antérieur on peut citer les produits de condensation des anhydrides polyalcénylsucciniques sur des polyamines, telles que, par exemple, la tétraéthylènepentamine, qui sont en particulier décrits dans le brevet USA-3 172 892. Ces additifs donnent de bons résultats au niveau des propriétés anticorrosion, mais ne sont pas efficaces comme détergents de soupapes.

On peut également citer les produits de condensation des anhydrides polyalcénylsucciniques sur des hydroxyimidazolines, et en particulier sur des 1-(2-hydroxyéthyl) imidazolines substituées en position 2 par un groupe alkyle ou alcényle, tels que ceux qui sont décrits dans la demande de brevet EP-A-74 724. Les produits décrits dans cette demande sont de bons additifs pour carburants moteurs et ont une action anticorrosion importante mais ne sont pas très efficaces au niveau de la détergence du carburateur.

L'encrassement des chambres de combustion se produit de façon progressive lors du fonctionnement du moteur. Ce dernier est caractérisé par son exigence en octane qui correspond au niveau minimum d'indice d'octane du carburant nécessaire au moteur afin de fonctionner sans cliquetis. Lorsque la valeur de l'exigence en octane du moteur excède, notamment par suite de l'encrassement des chambres de combustion, la valeur de l'indice d'octane du carburant utilisé pour alimenter ce moteur, on observe le phénomène de cliquetis. L'augmentation d'exigence en octane du moteur constitue classiquement, pour l'homme de l'art, le phénomène d'ORI d'après l'abréviation anglo-saxonne de "Octane Requirement Increase".

Afin de limiter l'apparition du cliquetis et ses conséquences néfastes sur le moteur telles que fatigue et usure accrues des parties vitales, il est possible de remédier à une trop forte exigence en octane du moteur en utilisant, sous réserve de disponibilité et à un coût économique élevé, un carburant ayant un indice d'octane supérieur à celui utilisé préalablement. On peut également procéder, de façon périodique, à un nettoyage des chambres de combustion afin d'éliminer les dépôts formés et réduire l'exigence en octane du moteur. Cette opération est toutefois longue et très coûteuse.

De très nombreux documents de brevets décrivent des additifs utilisables notamment dans les carburants moteurs. Les compositions telles que celles décrites par exemple dans la demande de brevet EP-A-327 097 ont de bonnes propriétés anti-ORI mais des propriétés détergentes relativement limitées. De plus ces compositions ne sont pas décrites comme ayant de bonnes propriétés anticorrosion.

On a maintenant découvert de façon surprenante des formulations, telles que décrites ci-après, utilisables notamment comme additifs multifonctionnels pour carburants moteurs en particulier pour les carburants utilisés dans les moteurs à allumage commandé. Les formulations de la présente invention ont d'excellentes propriétés détergentes au niveau des soupapes d'admission et du carburateur ou des injecteurs, et ont de très bonnes propriétés d'anticorrosion.

Les formulations selon la présente invention sont utilisables notamment comme additifs multifonctionnels pour carburants et, par exemple, comme additifs dans les carburants employés dans les moteurs à allumage commandé dans lesquels elles permettent, en particulier, de limiter l'augmentation d'exigence en octane (ORI) de ces moteurs et donc de limiter, de retarder ou même d'éviter, l'apparition du phénomène de cliquetis.

Les formulations selon la présente invention associent à cette action anti-ORI une action détergente aussi bien au niveau du carburateur, qu'au niveau des injecteurs ainsi qu'au niveau des soupapes d'admission. Par ailleurs, ces formulations possèdent des propriétés anticorrosion aussi bien dans les carburants employés pour les moteurs à allumage commandé que dans ceux utilisés dans les moteurs à allumage par compression (moteur Diesel). De plus, ces formulations utilisées dans les carburants employés pour les moteurs à allumage commandé permettent d'éviter, d'une part, l'apparition du phénomène de collage des soupapes d'admission et, d'autre part, ne contribuent pas à la formation de dépôts (boues noires) dans les huiles moteurs assurant la lubrification.

La présente invention a pour objet une formulation d'additifs, notamment pour carburants, qui comprend au moins un constituant (K), au moins un constituant (L) et au moins un constituant (M), ledit constituant (K) consistant en au moins un composé hétérocyclique de type imidazo-oxazole, comportant une chaîne latérale alkoxylée, de formule générale (I) : 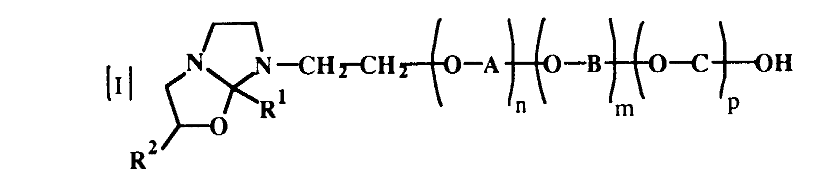 dans laquelle R¹ représente un atome d'hydrogène ou un groupement hydrocarboné ayant de 1 à 40 atomes de carbone et de préférence de 4 à 25 atomes de carbone, R² représente un atome d'hydrogène ou un groupement hydrocarboné ayant de 1 à 12 atomes de carbone, souvent de 1 à 6 atomes de carbone et de préférence de 1 à 3 atomes de carbone, A, B et C, identiques ou différents, représentent chacun un groupe hydrocarboné divalent ayant de 2 à 6 atomes de carbone et de préférence de 2 à 4 atomes de carbone, n est un nombre entier de 5 à 50 de préférence de 10 à 50 et le plus souvent de 10 à 25, m et p, identiques ou différents, sont chacun zéro ou un nombre entier de 1 à 25, et la somme n+m+p est un nombre entier de 1 à 50 de préférence de 5 à 30 et le plus souvent de 10 à 25, le dit constituant (L) consistant en au moins un composé choisi dans le groupe formé par les produits détergents-dispersants, et le dit constituant (M) consistant en au moins un composé choisi dans les huiles lubrifiantes minérales ou synthétiques et les polyglycols solubles dans ledit carburant.

A titre d'exemples de carburants pouvant contenir au moins une formulation d'additifs selon la présente invention, on peut citer les essences telles que celles qui sont définies par la norme ASTM D-439, les gazoles ou carburants Diesel tels que ceux qui sont définis par la norme ASTM D-975. Ces carburants peuvent également contenir d'autres additifs, tels que par exemple, notamment dans le cas des carburants employés pour les moteurs à allumage commandé, des additifs antidétonants tels que des composés du plomb (par exemple le plomb tétraéthyle), des éthers tels que le méthyltertiobutyléther ou le méthyltertioamyléther ou un mélange de méthanol et d'alcool tertiobutylique et des additifs antigivres. On peut également ajouter les formulations de la présente invention dans un carburant non hydrocarboné tel que par exemple un alcool ou un mélange d'alcools.

Le constituant (K) est le plus souvent choisi parmi les composés de formule générale (I) ci-devant dans laquelle R¹ représente un groupement alcoyle, alcényle, aryle, alkaryle ou aralkyle et de préférence un groupement alcoyle ou alcényle, linéaire ou ramifié, R² représente le plus souvent un atome d'hydrogène ou groupement alcoyle, linéaire ou ramifié et de préférence linéaire.

Les constituants (K) préférés, de formule générale (I) ci-devant, sont ceux dans lesquels m et p sont égaux à zéro, dans lesquels A représente un groupe alcoylène ayant de 2 à 4 atomes de carbone et R² un atome d'hydrogène lorsque A représente le groupe diméthylène de formule -CH₂-CH₂- et un groupement alcoyle de préférence linéaire ayant 2 atomes de carbone de moins que le groupement A lorsque celui-ci représente un groupement alcoylène ayant 3 ou 4 atomes de carbone. Le plus souvent les composés préférés sont ceux dans lesquels A représente le groupe diméthylène, le groupe méthyl-1 diméthylène ou le groupe éthyle-1 diméthylène qui sont des groupes dérivés respectivement de l'oxyde d'éthylène, de l'oxyde de propylène et de l'oxyde de butène-1 et qui ont les formules suivantes :

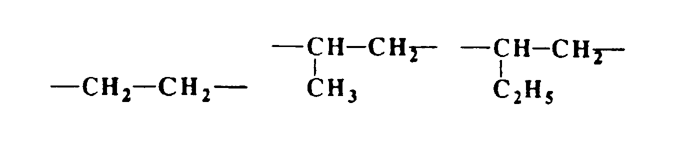

Les composés hétérocycliques de type imidazo-oxazoles utilisés comme constituant (K) peuvent être fabriqués par toutes méthodes connues de l'homme du métier. A titre d'exemples, non limitatifs, de méthodes permettant de préparer ces composés de formule générale (I) ci-devant on citera et on illustrera dans un exemple qui suit la méthode suivante.

Selon cette méthode on met en réaction, en présence ou en l'absence d'un solvant organique inerte, à une température de 90 °C à 190 °C et le plus souvent de 100 °C à 140 °C, en présence d'un catalyseur basique, au moins un oxyde d'alkylène ayant de 2 à 6 atomes de carbone et de préférence de 2 à 4 atomes de carbone dans sa molécule, avec au moins une 1-(2-hydroxyéthyl)-imidazoline non substituée ou substituée en position 2 par un groupement hydrocarboné ayant de 1 à 40 atomes de carbone de formule générale (II) suivante : 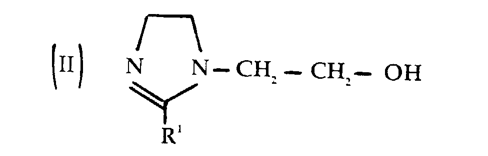 dans laquelle R¹ a la définition donnée ci-devant.

La réaction est habituellement effectuée sous une pression de 1 à 7 bar (1 bar est égal à 0,1 mégapascal). La durée de la réaction est variable suivant l'oxyde d'alkylène employé et également suivant le nombre de mole d'oxyde d'alkylène mise en oeuvre pour 1 mole d'imidazoline. Cette durée est souvent de 6 à 24 heures (h) et le plus souvent de 7 à 10 heures. Le rapport molaire imidazoline-oxyde l'alkyléne est habituellement de 1:5 à 1:50 et de préférence de 1:10 à 1:50 et le plus souvent de 1:10 à 1:25. On emploie habituellement une quantité de catalyseur basique de 0,1 à 0,6 mole par mole d'imidazoline. Le catalyseur est éliminé en fin de réaction soit par lavage à l'eau soit par un traitement de type échange d'ions par exemple à l'aide d'un solide échangeur d'ions.

Le catalyseur basique est habituellement choisi dans le groupe formé par le méthylate de sodium, l'éthylate de sodium, le tertiobutylate de potassium, la potasse et la soude.

L'oxyde d'alkylène est le plus souvent choisi dans le groupe formé par l'oxyde d'éthylène, l'oxyde de propylène et l'oxyde de butène-1.

Les imidazolines utilisées pour préparer les composés hétérocycliques de type imidazo-oxazoles de la présente invention sont des composés connus et pour certains d'entre eux commerciaux. Des imidazolines de formule (II) sont par exemple décrites dans la demande de brevet EP-A-74 724. Ces imidazolines sont le plus souvent synthétisées par réaction d'au moins un acide organique carboxylique sur la N-(2-hydroxyéthyl)-éthylènediamine. La réaction procède par une première étape d'amidification suivie d'une cyclisation. Les acides organiques utilisés ont habituellement de 2 à 26 atomes de carbone ; ce sont de préférence des acides aliphatiques monocarboxyliques.

A titre d'exemple on peut citer l'acide acétique, l'acide propanoique, l'acide butanoique, l'acide caproïque, l'acide caprique, l'acide laurique, l'acide myristique, l'acide palmitique, l'acide stéarique, l'acide béhénique, l'acide cérotique et les acides gras insaturés suivant :

| | |
|---|---|
| CH₃-CH₂-CH=CH-(-CH₂-)₇-COOH | acide dodécylènique |
| CH₃-(-CH₂-)₅-CH=CH-(-CH₂-)₇-COOH | acide palmitoléique |
| CH₃-(-CH₂-)₇-CH=CH-(-CH₂-)₇-COOH | acide oléique |
| CH₃-(-CH₂-)₅-CHOH-CH₂-CH=CH-(-CH₂-)₇-COOH | acide ricinoléique |
| CH₃-(-CH₂-)₁₀-CH=CH-(-CH₂-)₄-COOH | acide pétrosélénique |
| CH₃-(-CH₂-)₅-CH=CH-(-CH2-)₉-COOH | acide vaccénique |
| CH₃-(-CH₂-)₄-CH=CH-CH₂-CH=CH-(-CH₂-)₇-COOH | acide linoléique |
| CH₃-(-CH₂-)₉-CH=CH-(-CH₂-)₇-COOH | acide gadoléique |
| CH₃-(-CH₂-)₉-CH=CH-(-CH₂-)₉-COOH | acide cétoléique |
| CH₃-(-CH₂-)₇-CH=CH-(-CH₂-)₁₁-COOH | acide érucique |
| CH₃-(-CH₂-)₇-CH=CH-(-CH₂-)₁₃-COOH | acide sélacholéique |

On utilisera par exemple la 1-(2-hydroxyéthyl)-2-heptadécényl imidazoline, préparée par exemple à partir de l'acide oléique et de la N-(2-hydroxyéthyl)éthylènediamine. Cette préparation est par exemple décrite dans le brevet US-A-2 987 515. On peut également citer à titre d'exemple la 1-(2-hydroxyéthyl)-2-méthyl imidazoline préparée par exemple à partir de l'acide acétique et de la N-(2-hydroxyéthyl-)éthylènediamine. La 1-(2-hydroxyéthyl)-2 heptadécénylimidazoline est commercialisée par la société CIBA-GEIGY sous le nom "Amine-O".

Selon la présente invention les formulations comprendront au moins un constituant (L) choisi dans le groupe formé par les produits détergent-dispersants. Ce constituant (L) est habituellement choisi dans le groupe formé par les polyoléfines, de préférence les polyisobutènes, les polyisobutène-amines, les mélanges de ces types de composés et les produits qui sont en particulier décrits dans la demande de brevet européen EP-A-349 369 au nom de la demanderesse, ainsi que ceux décrits dans le brevet US-A-4 375 974. Les produits décrits dans la demande EP-A-349 369 résultent de la réaction dans une première étape d'au moins un dérivé succinique choisi dans le groupe formé par les acides et les anhydrides alcénylsucciniques et les acides et les anhydrides polyalcénylsucciniques sur au moins une 1-(2-hydroxy-éthyl-)imidazoline substituée en position 2 par un radical alkyle ou alcényle, linéaire ou ramifié, ayant de 1 à 25 atomes de carbone, le rapport molaire imidazoline/dérivé succinique étant de 0,1:1 à 0,9:1, de préférence de 0,2 : 1 à 0,8 : 1 et le plus souvent de 0,3 : 1 à 0,7:1, ladite étape étant effectuée dans des conditions telles que l'on forme et que l'on élimine au moins 0,15 mole d'eau par mole d'imidazoline engagée ; et dans une deuxième étape de la réaction du produit issu de la première étape sur au moins une polyamine répondant à l'une des formules générales suivantes : 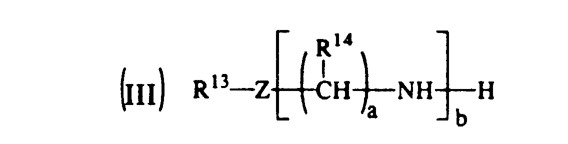 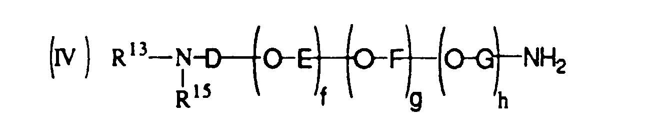 dans lesquelles R¹³ représente un atome d'hydrogène ou un groupe hydrocarboné ayant de 1 à 60 atomes de carbone, Z est choisi parmi les groupes -O-, et -NR¹⁵- dans lesquels R¹⁵ représente un atome d'hydrogène ou groupe hydrocarboné ayant de 1 à 60 atomes de carbone, R¹³ et R¹⁵ pouvant former ensemble avec l'atome d'azote auquel ils sont liés un hétérocycle, chacun des R¹⁴ indépendamment représente un atome d'hydrogène ou un groupe hydrocarboné ayant de 1 à 4 atomes de carbone, a est un nombre entier de 2 à 6, b est un nombre entier de 1 à 10 lorsque Z est -NR¹⁵- et un nombre entier de 2 à 10 lorsque Z est -O-, D, E, F et G, identiques ou différents, représentent chacun un groupe hydrocarboné divalent ayant de 2 à 6 atomes de carbone, f est un nombre entier de 1 à 60, g et h, identiques ou différents, sont chacun zéro ou un nombre entier de 1 à 50 et la somme f + g + h est un nombre entier de 1 à 60, la quantité de polyamine mise en réaction étant d'au moins 0,1 mole par mole de dérivé succinique introduit dans la première étape. La quantité totale d'imidazoline substituée et de polyamine est de préférence de 0,8 à 1,2 mole par mole de dérivé succinique.

L'acide ou le dérivé d'acide utilisé dans le cadre de la présente invention pour préparer le constituant (L) est habituellement un composé succinique ou un composé alkylsuccinique ou alcénylsuccinique de préférence un anhydride de type succinique. A titre d'exemple de composé de type succinique on peut citer l'anhydride succinique, l'anhydride méthylsuccinique souvent dénommé anhydride citraconique, et les anhydrides alkylsucciniques ou alcénylsucciniques ayant habituellement une masse moléculaire moyenne en nombre de 200 à 3 000, de préférence 500 à 2 000 et le plus souvent 700 à 1 500. Ces dérivés succiniques sont largement décrits dans l'art antérieur ; ils sont par exemple obtenus par l'action d'au moins une oléfine alpha ou d'un hydrocarbure chloré sur l'acide ou l'anhydride maléique. L'oléfine alpha ou l'hydrocarbure chloré utilisés dans cette synthèse peuvent être linéaires ou ramifiés, et comportent habituellement de 10 à 150 atomes de carbone, de préférence de 15 à 80 atomes de carbone et le plus souvent de 20 à 75 atomes de carbone dans leur molécule. Cette oléfine peut également être un oligomère, par exemple un dimère, un trimère ou un tétramère, ou un polymère d'une oléfine inférieure, ayant par exemple de 2 à 10 atomes de carbone, telle que l'éthylène, le propylène, le n-butène-l, l'isobutène, le n-hexène-l, le n-octène-l, le méthyl-2-heptène-1 ou le méthyl-2-propyl-5- hexène-1. Il est possible d'utiliser des mélanges d'oléfines ou des mélanges d'hydrocarbures chlorés.

A titre d'exemples d'anhydrides succiniques, on peut citer l'anhydride n-octadécénylsuccinique, l'anhydride dodécénylsuccinique et les anhydrides polyisobuténylsucciniques, souvent dénommés PIBSA, ayant une masse moléculaire moyenne en nombre telle que définie ci-devant.

Les 1-(2-hydroxyéthyl-)-imidazolines substituées en position 2 par un radical alkyle ou alcényle ayant de 1 à 25 atomes de carbone, utilisées dans le cadre de la présente invention pour préparer le constituant (L), sont habituellement des composés commerciaux ou qui peuvent être synthétisés par exemple par réaction d'au moins un acide organique avec la N-(2-hydroxy-éthyl)-éthylènediamine. La réaction procède comme décrit ci-devant et les acides employés sont ceux cités ci-devant dans le cadre de la préparation des composés de formule générale (I). On utilisera par exemple l'une des imidazolines citées ci-devant.

La première étape de préparation du constituant (L) est habituellement effectuée par addition progressive du dérivé de l'imidazoline à une solution du dérivé succinique dans un solvant organique, à température ordinaire, puis chauffage à une température habituellement comprise entre 65 °C et 250 °C et de préférence entre 80 °C et 200 °C. Le solvant organique utilisé dans cette préparation a un point d'ébullition compris entre 65 °C et 250 °C et est habituellement choisi manière à pouvoir permettre l'élimination de l'eau formée au cours de la condensation de l'imidazoline sur le dérivé succinique, de préférence sous forme d'un azéotrope eau-solvant organique. On utilisera habituellement un solvant organique tel que par exemple le benzène, le toluène, les xylènes, l'éthylbenzène ou une coupe d'hydrocarbures telle que par exemple la coupe commerciale SOLVESSO 150 (190-209 °C) contenant 99 % en poids de composés aromatiques. Il est possible d'utiliser des mélanges de solvants, par exemple un mélange de xylènes. La durée du chauffage après la fin de l'addition de l'imidazoline est habituellement de 0,5 à 7 heures, de préférence de 1 à 5 heures. Cette première étape sera de préférence poursuivie à la température choisie jusqu'à la fin du dégagement de l'eau formée au cours de la réaction.

La quantité d'eau éliminée au cours de cette première étape est habituellement de 0,15 à 0,6 mole et le plus souvent d'environ 0,5 mole par mole d'imidazoline engagée dans la réaction. Au produit ou mélange issu de cette première étape, après refroidissement éventuel, on ajoute de préférence progressivement au moins une polyamine, de préférence diluée dans un solvant organique, puis habituellement on chauffe à une température comprise entre 65 °C et 250 °C et de préférence entre 80 °C et 200 °C. Le solvant employé dans la deuxième étape est de préférence le même que celui qui est dans la première étape et la température est également la même au cours de ces deux étapes. Les réactions sont habituellement effectuées à une température correspondant à la température de reflux. La durée de ce chauffage au cours de cette deuxième étape est habituellement de 0,1 à 7 heures et de préférence de 0,2 à 5 heures. La quantité de polyamine employée est d'au moins 0,1 mole par mole d'anhydride succinique introduite au cours de la première étape et elle est de préférence telle que la quantité totale d'imidazoline substituée et de polyamine employée dans la préparation soit de 0,8 à 1,2 mole, de préférence de 0,9 à 1,1 mole par mole de dérivé succinique. Le rapport molaire imidazoline substituée sur polyamine est de préférence de 1 : 1 à 7 : 1 et de manière la plus préférée de 1 : 1 à 3 : 1.

La quantité d'eau éliminée au cours de cette deuxième étape est habituellement telle que la quantité d'eau totale éliminée au cours des deux réactions successives représente de 0,2 à 0,7 mole par mole de dérivé succinique.

Les polyamines de formule (III) sont de préférence celles dans lesquelles R¹³ est un atome d'hydrogène ou un groupe hydrocarboné ayant de 1 à 30 atomes de carbone, Z est de préférence un groupe -NR¹⁵- dans lequel R¹⁵ représente de préférence un atome d'hydrogène ou un groupe hydrocarboné ayant de 1 à 30 atomes de carbone, chacun des R¹⁴ indépendamment représente de préférence un atome d'hydrogène ou un groupe méthyle, a est un nombre entier de 2 à 4 et lorsque Z est un groupe -NR¹⁵- b est de préférence un nombre entier de 1 à 5.

Parmi les composés de formules (III) ci-devant on emploie avantageusement ceux dans lesquels Z est -NR¹⁵-, R¹³, R¹⁴ et R¹⁵ représentent chacun un atome d'hydrogène, a est égal à 2 et b est un nombre entier de 1 à 5 ou ceux dans lesquels R¹³ représente un groupe hydrocarboné ayant de préférence de 5 à 24 atomes de carbone, Z représente un groupe -NR¹⁵- dans lequel R¹⁵ est un atome d'hydrogène, R¹⁴ représente un atome d'hydrogène, a est un nombre entier de 2 à 4, de préférence 3, et b est un nombre entier de 1 à 5, de préférence 1.

Les groupes hydrocarbonés R¹³ et R¹⁵ sont habituellement des groupes alkyles, alcényles, linéaires ou ramifiés, aryles, aryl-alkyles (aralkyles), alkyl-aryles (alkaryles) ou cycloaliphatiques. Les groupes R¹³ et R¹⁵ sont de préférence des groupes alkyles ou alcényles, linéaires ou ramifiés. Le groupe hydrocarboné R¹⁴ est habituellement un groupe alkyle de préférence linéaire et par exemple méthyle, éthyle, n-propyle ou n-butyle.

Comme composés spécifiques on peut citer : les diamines alpha-oméga biprimaires mentionnées ci-après, la triméthylènediamine, les triméthyl-2,2,4 et -2,4,4 hexaméthylènediamine, les N-alkyl diamino-1,3 propane par exemple le N-dodécyldiamino-1,3 propane, le N-tétradécyldiamino-1,3 propane, le N-hexadécyldiamino-1,3 propane, le N-octadécyldiamino-1,3 propane, le N-éicosyldiamino-1,3 propane et le N-docosyldiamino-1,3 propane ; on peut également citer les N-alkyldipropylène triamines par exemple la N-héxadécyldipropylène triamine, la N-octadécyldipropylène triamine, la N-éicosyldipropylène triamine et la N-docosyldipropylène triamine ; on peut également citer les N-alcényldiamino-1,3 propane et les N-alcényldipropylène triamines par exemple le N-octadécényldiamino-1,3 propane, le N-héxadécényldiamino-1,3 propane, le N-dodécylényldiamino-1,3 propane, le N-octadécadiényldiamino-1,3 propane et le N-docosényldiamino-1,3 propane. On peut citer à titre d'exemples de diamines N,N disubstituées le N,N-diéthyl diamino-1,2 éthane, le N,N-diisopropyl diamino-1,2 éthane, le N,N-dibutyl diamino-1,2 éthane, le N,N-diéthyl diamino-1,4 butane, le N,N-diméthyl diamino-1,3 propane, le N,N-diéthyl diamino-1,3 propane, le N,N-dioctyl diamino-1,3 propane, le N,N-didécyl diamino-1,3 propane, le N,N-didodécyl diamino-1,3 propane, le N,N-ditétradécyl diamino-1,3 propane, le N,N-dihexadécyl diamino-1,3 propane, le N,N-dioctadécyl diamino-1,3 propane, la N,N-didodécyldipropylène triamine, la N,N-ditétradécyldipropylène triamine, la N,N-dihexadécyldipropylène triamine, la N,N-dioctadécyldipropylène triamine, le N-méthyl, méthyl, N-butyl diamino-1,2 éthane, le N-méthyl N-octyl diamino-1,2 éthane, le N-octyl diamino-1,2 éthane, le N-méthyl, N-décyl diamino-1,2 éthane, le N-méthyl N-dodécyl diamino 1,3 propane, le N-méthyl, N-hexadécyl diamino-1,3 propane et le N-éthyl N-octadécyl diamino-1,3 propane.

A titre d'exemples d'étheramines on peut citer le N-(octyloxy-3 propyl)diamino-1,3 propane, le N-(décyloxy-3 propyl)diamino-1,3 propane, le N- [(triméthyl-2,4,6 décyl)oxy-3 propyl] diamino-1,3 propane.

Il doit être entendu qu'il est possible de mettre en jeu comme composé polyaminé un ou plusieurs composés répondant à la formule (III) et/ou (IV). Comme exemples spécifiques de mélange de composés répondant à la formule (III) on peut citer :
les coupes de diamines grasses répondant à la formule R¹³-NH-(-CH₂-)₃-NH₂ dont les groupes R¹³ sont des radicaux hydrocarbonés aliphatiques en C₈, C₁₀, C₁₂, C₁₄, C₁₆, C₁₈, C₂₀ et C₂₂, en proportions molaires approximatives données dans le tableau I ci-après.

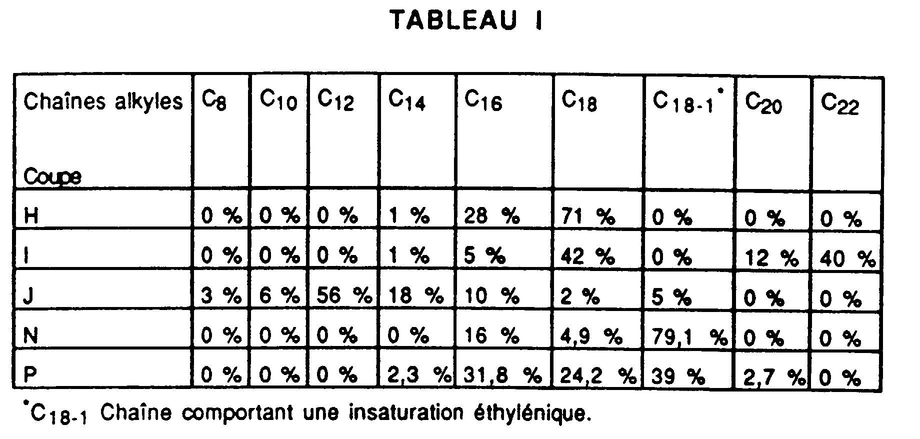

Les polyamines de formules (IV) sont de préférence celles dans lesquelles R¹³ et R¹⁵ représentent chacun un atome d'hydrogène, D, E, F et G, identiques ou différents, représentent chacun un groupe alkylène ayant de 2 à 4 atomes de carbone par exemple éthylène, triméthylène, méthyléthylène, tétraméthylène, méthyltriméthylène, méthyl-1 triméthylène et méthyl-2 triméthylène, f est un nombre entier de 1 à 60 et g et h sont égaux à zéro ou f est un nombre entier de 1 à 59, h est zéro ou un nombre entier tel que la somme f + h soit de 1 à 59 et g est un nombre entier de 1 à 50, avec dans chaque cas la somme f + g + h égale à un nombre entier de 1 à 60.

Comme composés spécifiques de formule (IV) on peut citer les diamines alpha-oméga biprimaires qui sont des composés bien connus de l'homme du métier. Comme composés spécifiques on peut citer, à titre d'exemple non limitatif, : l'éthylènediamine, la propylènediamine, la diéthylènetriamine, la dipropylènetriamine, la triéthylènetétramine, la tripropylènetétramine, la tétraéthylènepentamine, la tétrapropylènepentamine, l'hexaméthylènediamine, la di(triméthylène)-triamine, la diméthyl-2,2 propanediamine-1,3, la N,N'-bis(amino-3 propyl)-éthylènediamine, la (amino-2 éthyl)-amino-3 propylamine, les triméthyl-hexaméthylènediamines pour le cas des amines ne contenant pas d'atomes d'oxygène dans leur formule. Dans le cas d'amines contenant des atomes d'oxygène dans leur formule on peut citer les polyamines de formule :

N H₂-R⁸-(-O-R⁹-)_{c}-(-O-R¹⁰-)_{d}-(-O-R¹¹-)ₑ-NH₂

dans laquelle de préférence R⁸, R⁹ R¹⁰ et R¹¹, identiques ou différents, représentent chacun un groupe alkylidène ayant de 2 à 4 atomes de carbone par exemple éthylidène (diméthylène), propylidène (triméthylène), isopropylidène (méthyl-1 diméthylène), butylidène (tétraméthylène), isobutylidène (méthyl-2 triméthylène), de préférence c est un nombre entier de 1 à 60 et d et e sont égaux à zéro ou c est un nombre entier de 1 à 59, e est zéro ou un nombre entier tel que la somme c +e soit de 1 à 59 et d est un nombre entier de 1 à 50, avec dans chaque cas la somme c+ d + e égale à un nombre entier de 1 à 60.

Comme diamines spécifiques on peut encore citer celles répondant aux formules (V), (VI) et (VII) suivantes : 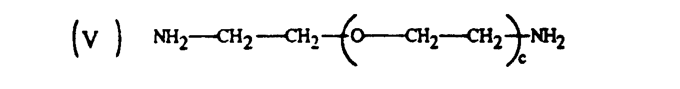 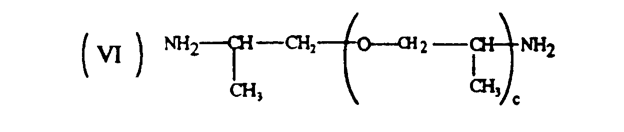 dans lesquelles c est 2, 3, 5, 6 ou 33, ou de formule : 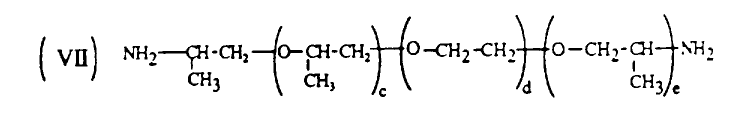 dans laquelle d est égal à 8, 9, 15, 16 ou 40 et c + e est 2 ou 3.

Ces produits sont en particulier commercialisés par la société TEXAC0 Chemical sous le nom Jeffamine EDR 148 pour le produit de formule (V) dans laquelle c = 2, Jeffamine D-230 pour un produit de formule (VI) de masse moléculaire moyenne en nombre de 230, Jeffamine D-400 pour un produit de formule (VI) de masse moléculaire moyenne en nombre de 400, Jeffamine D-2 000 pour un produit de formule (VI) de masse moléculaire moyenne en nombre de 2 000, Jeffamine ED-600 pour un produit de formule (VII) de masse moléculaire moyenne en nombre de 600, Jeffamine ED-900 pour un produit de formule (VII) de masse moléculaire moyenne en nombre de 900 et Jeffamine ED-2 001 pour un produit de formule (VII) de masse moléculaire moyenne en nombre de 2 000. Dans le cadre de la présente invention on peut utiliser, pour la synthèse des produits, une ou plusieurs diamines biprimaires.

Les produits décrits par la demanderesse dans le brevet US-A-4 375 974 et utilisables, dans le cadre de la présente invention comme constituant (L) sont ceux résultant de la réaction d'au moins une polyamine, ayant au moins un groupe amino primaire et répondant à la formule générale (III) ci-devant, sur au moins un dérivé succinique tels que ceux décrits ci-devant, ladite réaction étant effectuée dans des conditions de formation et d'élimination de l'eau de réaction. Le plus souvent la réaction est effectuée à une température de 120 °C à 200 °C avec un rapport molaire amine sur dérivé succinique de 0,9 : 1 à 1,2 : 1. Cette réaction peut être effectuée en l'absence de solvant ou en présence d'un solvant tel que par exemple un hydrocarbure aromatique ou une coupe d'hydrocarbures ayant un point d'ébullition de 70 °C à 250 °C.

Le constituant (L) utilisable dans le cadre de la présente invention peut aussi être choisi dans le groupe formé par les polyisobutènes, les polyisobutène-amines, les mélanges de ces deux types de composés. Les polyoléfines employées peuvent être des polymères ou des copolymères ou les dérivés aminés ou hydrogénés correspondants formés à partir d'hydrocarbures ayant de 2 à 10 atomes de carbone dans leur molécule.

Ces composés polymériques sont habituellement préparés à partir de composés mono-oléfiniques ou dioléfiniques et ont habituellement une masse moléculaire moyenne en nombre de 500 à 10 000 souvent de 500 à 3 500 et de préférence de 650 à 2 600. Le plus souvent les composés de départ employés pour fabriquer ces polymères sont des oléfines ayant de 2 à 6 atomes de carbone dans leur molécule, telles que par exemple l'éthylène, le propylène, l'isopropylène, le butène, l'isobutène, l'amylène, l'héxylène, le butadiène et l'isoprène. On utilise très fréquemment le propylène, l'isopropylène, le butène et l'isobutène. Les autres polyoléfines qui peuvent également être employées sont celle obtenues par craquage de polymères ou de copolymères oléfiniques de poids moléculaire élevés en composés ayant une masse moléculaire dans la gamme de poids moléculaire mentionnée ci-devant.

A titre d'exemples non limitatifs de composés spécifiques que l'on utilise fréquemment on peut citer les polypropylènes de masse moléculaire moyenne en nombre de 750 à 1 000 et par exemple d'environ 800, les polyisobutènes de masse moléculaire moyenne en nombre de 1 000 à 1 500 et par exemple d'environ 1 300.

Dans une autre réalisation préférée selon la présente invention le constituant (L) est un mélange comprenant une proportion majoritaire de polyisobutène-éthylènediamine et une proportion minoritaire de polyisobutène. Ce mélange est le plus souvent employé dissous dans un solvant hydrocarboné de manière à faciliter son incorporation dans le carburant. La proportion de polymère aminé au sein de ce mélange est habituellement de 50 % à 80 % en poids et par exemple d'environ 60 % en poids et la proportion de polymère hydrocarboné est habituellement de 5 % à environ 30 % en poids et de préférence de 10% à 25 % en poids.

La polyisobutène éthylène diamine est un composé de formule générale : 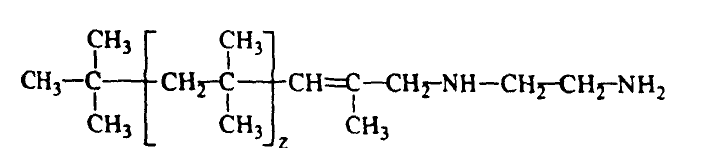 dans laquelle z est un nombre de 10 à 40, de préférence de 30 à 35 et par exemple 33.

Le polyisobutène est un composé de formule générale : 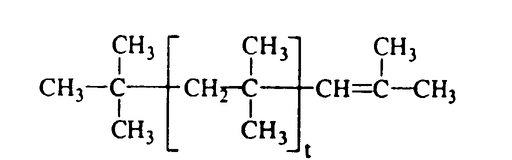 dans laquelle t est un nombre de 10 à 40, de préférence de 30 à 35 et par exemple 33.

Le solvant employé pour dissoudre les composés polymèriques et faciliter leur incorporation au carburant est le plus souvent un distillat aromatique léger. On peut employer en tant que constituant (L) comprenant, dissous dans un distillat aromatique léger, un polyisobutène et un polyisobutène-éthylène-diamine tels que décrits ci-devant, le produit vendu par la société CHEVRON CHEMICAL COMPANY sous le nom commercial ORONITE OGA-472. L'ORONITE OGA-472 est une composition comprenant approximativement 60 % en poids de polyisobutène-éthylène-diamine, approximativement 27 % en poids de polyisobutène et approximativement 13 % en poids de distillat aromatique léger comprenant du xylène et des alkylbenzènes en C₉.

Selon la présente invention les formulations contiennent également au moins un constituant (M) choisi dans le groupe formé par les huiles lubrifiantes minérales ou synthétiques et les polyglycols, solubles dans ledit carburant, ayant de préférence une masse moléculaire moyenne en nombre de 480 à 2 100 et de formule générale (VIII) : 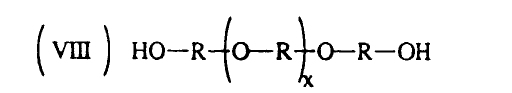 dans laquelle chacun des groupes R indépendamment représente un groupe hydrocarboné ayant de 2 à 6 atomes de carbone et x représente le degré moyen de polymérisation. Ces polyglycols sont par exemple ceux décrits par la demanderesse dans la demande de brevet européen EP-A-349 369.

Dans une forme avantageuse de réalisation le constituant (M) est un polyglycol, ayant un indice de polydispersité de 1 à 1,25 et de préférence de 1 à 1,15, de formule générale (VIII) dans laquelle chacun des groupes R indépendamment représente un groupe alkylène, linéaire ou ramifié, ayant de 2 à 4 atomes de carbone, de préférence un groupe éthylène ou propylène.

Parmi les polyglycols de formule générale (VIII) particulièrement préférés, on peut citer ceux dans lesquels chacun des groupes R représente un groupe propylène de formule :

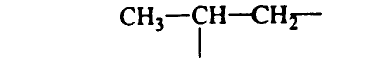

Le polyglycol employé est de préférence un polyglycol de masse moléculaire moyenne en nombre de 600 à 1 800 et le plus souvent de 650 à 1 250.

Parmi les huiles lubrifiantes minérales ou synthétiques que l'on peut employer comme constituant (M) on peut citer à titre d'exemples non limitatifs pour les huiles minérales, les huiles lubrifiantes connues par l'homme du métier sous les dénominations 500 NS et 600 NS, et pour les huiles lubrifiantes synthétiques les éthers et les esters de polyols et en particulier les éthers de polyoxyalkylèneglycols.

Les formulations selon l'invention sont en particulier utilisables comme additif ayant une bonne activité anticorrosion pour un carburant à base d'hydrocarbures ou d'un mélange d'hydrocarbures et d'au moins un composé oxygéné choisi dans le groupe formé par les alcools et les éthers. Ces formulations sont en outre utilisables comme additif multifonctionnel ayant en particulier de bonnes propriétés anti-ORI et détergente-dispersantes pour un carburant moteur, pour moteur à allumage commandé, à base d'hydrocarbures ou d'un mélange d'hydrocarbures et d'au moins un composé oxygéné choisi dans le groupe formé par les alcools et les éthers. Habituellement ces formulations sont ajoutées au carburant de manière à obtenir une concentration en masse, de la composition d'additif dans le carburant moteur, de 10 à 10 000 ppm, souvent de 100 à 5 000 ppm et de préférence de 100 à 2 000 ppm.

Dans les formulations selon la présente invention le rapport pondéral du constituant (K) au constituant (L) [ (K)/(L) ] est habituellement de 0,02 : 1 à 4 : 1.

Ce rapport est souvent de 0,02 : 1 à 2 : 1 et de préférence, de 0,1 : 1 à 2 : 1.

Le rapport pondéral du constituant (L) au constituant (M) [ (L)/(M) ] est habituellement de 0,05 : 1 à 10 : 1. Ce rapport est souvent de 0,05 : 1 à 5 : 1 et, de préférence, de 0,01 : 1 à 2 : 1.

L'invention porte également sur les composés hétérocycliques de type imidazo-oxazole alkoxylés eux-mêmes, ayant la formule (I), tels qu'ils ont été décrits précédemment.

Les exemples qui suivent illustrent l'invention sans en limiter la portée.

### Exemple 1

Dans un réacteur de 1 litre, équipé d'un système d'agitation mécanique, d'un système de mesure et de régulation de température ainsi que d'un condenseur refroidi par un mélange acétone-glace carbonique, on introduit 106 grammes (g) (0,5 mole) de 1-(2-hydroxyéthyl)-2-héptylimidazoline et 10,8 g (0,16 mole) de méthylate de sodium. On porte ensuite ce mélange à 120 °C sous agitation pendant 1 heure (h) sous un vide de 13,33 hPa (10 millimètres de mercure). Après rétablissement de la pression atmosphérique, on procède à l'addition lente de 493 g (8,5 moles) d'oxyde de propylène en 8 heures et 30 minutes. Le milieu réactionnel est maintenu à 120 °C pendant toute la durée d'addition, puis pendant encore 30 minutes après la fin de cette addition avant d'être refroidi à température ambiante d'environ 20 °C.

Le produit obtenu est transvasé dans un erlenmeyer surmonté d'un réfrigérant puis dilué avec 300 ml de toluène. Après ajout de 89 g de résine Ambosol 500 (nom commercial d'un silicate acide de magnésium vendu par la société HOECHST), le milieu est porté à 75 °C pendant 1 heure sous agitation. Après filtration et évaporation du solvant on obtient 563 g (soit une conversion d'environ 93 %) du produit final recherché. La viscosité cinématique de ce produit mesurée à 40 °C est de 1,68 10⁻⁴ m²/s. Ce produit est caractérisé par infrarouge, spectrométrie de masse et analyse élémentaire. En infrarouge on observe l'absence de la bande d'absorption intense à 1 550-1 600 cm⁻¹ qui est caractéristique de la double liaison carbone-azote du cycle imidazoline. Ce produit, de formule générale (I) dans laquelle R¹ représente le groupement heptyl de formule brute -C₇H₁₅, R² représente le groupement méthyl, A représente le groupement méthyl-1 diméthylène, m et p sont égaux à zéro et n est égal à 16, a une masse moléculaire calculée de 1 199,7. L'analyse élémentaire calculée et mesurée est :

| Elément | % mesuré | % calculé |
|---|---|---|
| H | 11,1 | 10,59 |
| C | 62,05 | 63,07 |
| N | 2,12 | 2,34 |
| O | | 24,01 |

Le spectre de masse obtenu par impact électronique sur un appareil à secteur magnétique ayant un faisceau électronique d'une puissance de 70 électron-volt présente un fragment de masse moléculaire 59 de formule : 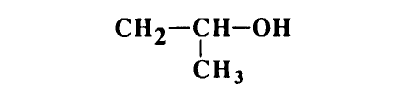 un fragment de masse moléculaire 117 et un fragment de masse moléculaire 175 de formule : 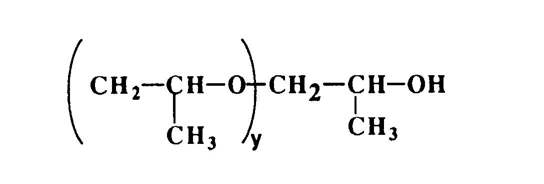 dans laquelle y est respectivement 1 et 2 et un fragment de masse moléculaire 239 de formule :

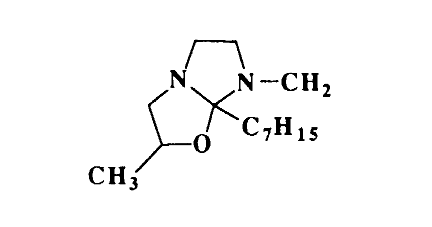

### Exemple 2 (Composition détergente)

1 018 g d'anhydride polyisobuténylsuccinique (PIBSA), résultant de la condensation de polyisobutène (polyisobutène de masse moléculaire moyenne en nombre de 920), sur l'anhydride maléique (le dosage des fonctions anhydride de ce produit montre que l'on a 0,7 fonction anhydride par kilogramme) et 1 018 g de xylène sont chargés dans un réacteur de 2 litres muni d'une agitation mécanique, d'un séparateur de Dean-Stark et d'un système de régulation de température.

On procède ensuite, à température ambiante et sous agitation, à l'addition goutte à goutte de 148 g (0,423 mole) de 1-(2-hydroxy-éthyl)-2-heptadécényl-imidazoline diluée dans 148 g de xylène. L'addition est effectuée en 30 minutes et accompagnée d'une augmentation rapide de température du mélange réactionnel d'environ 5°C. Le mélange est ensuite porté à reflux pendant 3 heures avec élimination d'eau réactionnelle par distillation azéotropique. La quantité d'eau recueillie est de 2,3 ml (millilitre) L'état d'avancement de la réaction peut également être suivi par spectrométrie infrarouge au niveau de la bande d'absorption de la fonction imine à 1 660 cm⁻¹ qui disparaît progressivement au cours de la réaction.

La température du réacteur est diminuée jusqu'à 50 °C puis maintenue à cette valeur durant le temps de l'addition progressive (goutte à goutte) de 56 g (0,297 mole) de tétraéthylènepentamine diluée dans 49 g de xylène. A la fin de cette addition le mélange est de nouveau porté à reflux pendant 15 minutes. Il se produit de nouveau une élimination d'eau. La quantité totale d'eau recueillie au cours de ces deux étapes de réaction est de 7,2 ml. Le spectre infrarouge montre deux bandes d'absorption (1 710 cm⁻¹ et 1 770 cm⁻¹) caractéristique de la fonction succinimide avec un épaulement (1 740 cm⁻¹) caractéristique de la fonction ester.

On obtient ainsi une solution, à 50 % en poids de matière active, dans le xylène, d'une composition dont l'analyse élémentaire fait apparaître une teneur en azote de 2,55 % en poids.

### Exemple 3

On prépare des solutions, dans le xylène, de formulations F1 à F7 comprenant diverses quantités pondérales des constituants K, L, M définis ci-après.

Le constituant K est formé par le produit obtenu dans l'exemple 1.

Le constituant L est formé par la composition obtenue dans l'exemple 2.

Le constituant M est un polypropylèneglycol de formule : 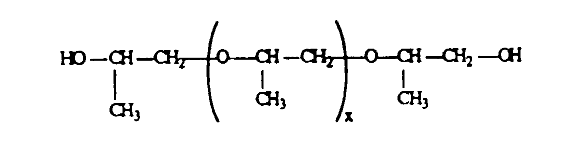 dont la masse moléculaire moyenne en nombre est de 922 (x = 13,6) et dont la polydispersité est de 1,1.

La formulation F1 selon la présente invention contient le constituant K obtenu dans l'exemple 1, le constituant L et le constituant M décrits ci-devant. Ces constituants sont utilisés dans un rapport pondéral, en terme de matière active, K : L : M de 1 : 5 : 4.

La formulation F2 (formulation de comparaison) contient le constituant L ainsi que le constituant M décrits ci-devant, mais pas de constituant K. Le rapport pondéral en matière active L : M est de 1,25 : 1.

La formulation F3 (formulation de comparaison) contient le constituant L décrit ci-devant, mais pas de constituant K ni M.

La formulation F4 (formulation de comparaison) contient le constituant M décrit ci-devant, mais pas de constituant K ni L.

La formulation F5 (formulation de comparaison) contient le constituant K ainsi que le constituant L décrits ci-devant, mais pas de constituant M. Le rapport pondéral en matière active K : L est de 0,2 : 1.

La formulation F6 (formulation de comparaison) contient le constituant K ainsi que le constituant M décrits ci-devant, mais pas de constituant L. Le rapport pondéral en matière active K : M est de 0,25 : 1.

La formulation F7 (formulation de comparaison) contient le constituant K décrit ci-devant, mais pas de constituant L ni M.

### Exemple 4

Une série d'essais est effectuée de manière à évaluer les propriétés de limitation de l'augmentation d'exigence en octane d'un moteur alimenté avec un carburant seul, et avec un carburant contenant l'une des formulations décrites dans l'exemple 3.

Le carburant (S1)utilisé est un carburant sans plomb comprenant en volume :
- 16 % d'aromatiques
- 14 % d'oléfines
- 59,6 % de composés saturés (paraffines et naphténiques)
- 10,4 % de méthyltertiobutyléther

Les diverses formulations ont été ajoutées au carburant de manière à avoir une concentration en pois de matière active de 600 ppm.

Les essais ont été effectués sur un banc moteur équipé d'un moteur Renault de type F3N ayant une cylindrée de 1 721 cm³ et un taux de compression de 9,5. Ces essais ont été effectués en suivant la procédure Renault 22 700, avec une température d'eau en sortie de culasse de 95 °C à plus ou moins 2 degrés et une température de l'huile au niveau de la rampe de 140 °C. Le cycle d'essai dure 12 heures (h) et comprend :
- 1 h de ralenti à vide
- 4 h à 2 500 tours par minute (t/mn) à la moitié de la pleine charge
- 3 h à 3 500 t/mn à vide
- 4 h à 2 500 t/mn à la moitié de la pleine charge.

Les valeurs d'avance à l'allumage, correspondant à l'apparition du phénomène de cliquetis, exprimées en degrés vilebrequin et désignées par les initiales KLSA (initiales anglo-saxonnes de " Knock Limit Spark Advance", sont déterminées successivement à 0, 50, 100, 150, 200, 300 et 400 heures pour 7 régimes différents du moteur : 1 500, 2 000, 2 500, 3 000, 3 500, 4 000 et 4 500 t/mn. Les résultats obtenus sont exprimés en terme de variations d'avance limite à l'allumage entre 0 et 400 heures (Δ KLSA) aux différents régimes moteur et sont présentés dans le tableau II ci-après. Les poids moyens (exprimés en milligramme (mg) par soupape) des dépôts sur les soupapes d'admission sont également mentionnés dans ce tableau.

Ces résultats montrent que le carburant contenant la formulation selon l'invention donne des valeurs plus faibles de Δ KLSA que le carburant seul ou que le carburant contenant la majorité des formulations de comparaison et limite ainsi l'augmentation de l'exigence en octane du moteur ce qui retarde l'apparition du cliquetis. On constate également que la formulation selon l'invention permet de réduire le poids des dépôts sur les soupapes d'admission par rapport à ce que l'on obtient avec le carburant seul ou avec le carburant contenant la majorité des formulations de comparaison.

Par ailleurs, on remarque que le carburant contenant la formulation F7 est efficace au niveau de la limitation de l'augmentation de l'exigence en octane du moteur mais n'est pas efficace pour limiter le poids de dépôts sur les soupapes d'admission.

**TABLEAU II**

| Δ KLSA (°V) à 400 h | *carburant seul(S1) | S1 +F1 | *S1 +F2 | *S1 +F3 | *S1 +F4 | *S1 +F5 | *S1 +F6 | *S1 +F7 |
|---|---|---|---|---|---|---|---|---|
| Régime moteur t/mn | | | | | | | | |
| 1 500 | 8 | 5 | 8 | 9 | 8 | 7 | 7 | 6 |
| 2 000 | 10 | 6 | 9 | 10 | 9 | 8 | 8 | 7 |
| 2 500 | 12 | 9 | 11 | 13 | 11 | 10 | 9 | 9 |
| 3 000 | 13 | 8 | 13 | 13 | 13 | 9 | 11 | 9 |
| 3 500 | 11 | 5 | 11 | 12 | 10 | 7 | 8 | 6 |
| 4 000 | 9 | 4 | 8 | 10 | 8 | 6 | 7 | 5 |
| 4 500 | 7 | 5 | 7 | 8 | 7 | 7 | 6 | 4 |
| Dépôts soupapes mg/soupape | 103 | 8 | 8 | 15 | 110 | 76 | 92 | 149 |

| | | | | | | | | |
|---|---|---|---|---|---|---|---|---|
| *Comparaison | | | | | | | | |

### Exemple 5

On procède à l'évaluation des propriétés de détergence "caburateur" des carburants contenant les formulations préparées dans l'exemple 3. La procédure d'essai moteur est effectuée en suivant la norme européenne R5-CEC-F03-T-81. Les résultats sont exprimés en terme de mérite de zéro à dix. Un mérite 10 correspond à un carburateur propre et un mérite 0 à un carburateur très encrassé. Les formulations sont ajoutées au carburant de manière à obtenir une concentration en poids de matière active dans le carburant additivé précisée pour chaque exemple dans le tableau III ci-après qui donne les résultats obtenus :

**TABLEAU III**

| CARBURANT ADDITIVE | QUANTITE ADDITIF | MERITE |
|---|---|---|
| *carburant seul | 0 ppm | 3,31 |
| carburant + formulation F1 | 600 ppm | 9,25 |
| *carburant + formulation F2 | 600 ppm | 9,18 |
| *carburant + formulation F3 | 600 ppm | 9,12 |
| *carburant + formulation F4 | 600 ppm | 3,82 |
| *carburant + formulation F5 | 600 ppm | 9,15 |
| *carburant + formulation F6 | 600 ppm | 9,01 |
| *carburant + formulation F7 | 600 ppm | 9,07 |

| | | |
|---|---|---|
| *Comparaison | | |

Le carburant utilisé dans ces évaluations est un supercarburant (S2), sans plomb, d'indice d'octane moteur de 85,8 et d'indice d'octane recherche de 96,4. Ce supercarburant a un point initial de distillation de 35 °C et un point final de distillation de 204 °C.

Ce supercarburant (S2) comprend en volume :
- 55,6 % de composés saturés (paraffines + naphténiques)
- 10,8 % d'oléfines
- 33,6 % d'aromatiques.

### Exemple 6

Une série d'essais est effectuée de manière à évaluer les propriétés de détergence-soupape de diverses formulations. Les essais ont été réalisés sur banc moteur Mercédes M102E, sans additif et avec addition d'additifs dans le carburant. La procédure d'essai est une procédure classique comprenant l'utilisation d'un moteur ayant 4 cylindres, de type Mercédes M102E, ayant une cylindrée de 2 299 cm³ et un taux de compression de 9/1. La procédure de test est une procédure cyclique, chaque cycle comprenant quatre périodes successives de fonctionnement :
- 30 s (secondes) à 800 t/min (tours par minute) sous une charge nulle,
- 60 s à 1 300 t/min sous une charge de 31 newtons (m x kg x s⁻²)
- 120 s à 1 850 t/min sous une charge de 34 newtons et
- 60 s à 3 000 t/min sous une charge de 37 newtons.

La durée de chaque test est habituellement de 60 heures. Au départ de chaque test, le moteur est conditionné avec des soupapes neuves que l'on pèse. En fin d'essai, les soupapes sont démontées, lavées à l'hexane, séchées, puis pesées après élimination physique (par grattage) des dépôts formés sur la soupape côté chambre de combustion. Les résultats présentés ci-après donnent la moyenne des dépôts en poids rapportée à une soupape, calculée à partir du poids de dépôts mesuré, sur la tulipe de chaque soupape d'admission, par différence entre le poids de ladite soupape neuve et le poids de ladite soupape à la fin de chaque essai après élimination des dépôts côté chambre de combustion. On évalue également par cotation visuelle l'état de chaque soupape (côté admission : tulipe) en terme de mérite de 1 à 10 selon la procédure habituellement dénommée CRC (initiales anglaises de Coordinating Research Council) par les hommes du métier ; les résultats sont exprimés ci-après sous forme de moyenne par soupape ; un mérite de 10 correspond à une soupape propre et un mérite de 1 à une soupape très encrassée.

Le carburant utilisé dans ces évaluations est le supercarburant sans plomb (S2) dont les principales caractéristiques ont été données dans l'exemple 5 ci-devant.

Les formulations sont ajoutées au carburant de manière à obtenir une concentration, en poids de matière active dans le carburant additivé, précisée pour chaque exemple dans le tableau IV ci-après donnant les résultats obtenus :

**TABLEAU IV**

| Carburant | Quantité Additif | Moyenne des Dépôts en mg | Moyenne CRC |
|---|---|---|---|
| *Carburant seul (S2) | 0 ppm | 337 | 8,11 |
| S2 + F1 | 600 ppm | 42 | 9,44 |
| *S2 + F2 | 600 ppm | 48 | 9,40 |
| *S2 + F3 | 600 ppm | 121 | 8,53 |
| *S2 + F4 | 600 ppm | 343 | 8,05 |
| *S2 + F5 | 600 ppm | 152 | 8,47 |
| *S2 + F6 | 600 ppm | 171 | 8,32 |
| *S2 + F7 | 600 ppm | 329 | 8,10 |

| | | | |
|---|---|---|---|
| * Comparaison | | | |

### Exemple 7

On procède à l'évaluation des propriétés anticorrosion des formulations préparées dans l'exemple 3. Les essais consistent à déterminer l'étendue de la corrosion produite sur des échantillons d'acier ordinaire poli, en présence d'eau, en suivant la norme ASTM D 665 modifiée (température 32,2 °C, durée 20 heures). Les résultats sont exprimés en pourcentage (%) de la surface de l'éprouvette corrodée au bout de 20 heures. Le carburant utilisé est un carburant Diesel dont les principales caractéristiques sont les suivantes :
- Température limite de filtrabilité : - 4 °C
- Point initial de distillation : 160 °C
- Point 95 % de distillation : 370 °C
- Masse volumique à 15 °C : 0,84
- Indice de cétane calculé : 52

La quantité de formulation est ajoutée au carburant de manière à obtenir une concentration, en poids de matière active dans le carburant additivé, précisée pour chaque exemple dans le tableau V ci-après donnant les résultats obtenus :

**TABLEAU V**

| **CARBURANT ADDITIVE** | **QUANTITE ADDITIF** | **% DE SURFACE CORRODEE** |
|---|---|---|
| *Carburant seul | 0 ppm | 100 |
| Carburant + Formulation F1 | 600 ppm | 0 |
| *Carburant + Formulation F2 | 600 ppm | 0 |
| *Carburant + Formulation F3 | 600 ppm | 0 |
| *Carburant + Formulation F4 | 600 ppm | 100 |
| *Carburant + Formulation F5 | 600 ppm | 50 |
| *Carburant + Formulation F6 | 600 ppm | 100 |
| *Carburant + Formulation F7 | 600 ppm | 100 |

| | | |
|---|---|---|
| *Comparaison | | |

L'analyse des résultats obtenus dans les exemples précédents montre que les formulations selon la présente invention permettent de limiter très significativement l'augmentation d'exigence en octane des moteurs à allumage commandé et possède des qualités d'additifs détergents du système d'admission ainsi que d'anticorrosion.

## Revendications

1. Formulation d'additifs, notamment pour carburants, caractérisée en ce qu'elle comprend au moins un constituant (K), au moins un constituant (L) et au moins un constituant (M), ledit constituant (K) consistant en au moins un composé hétérocyclique de type imidazo-oxazole, comportant une chaîne latérale alkoxylée, de formule générale (I) : 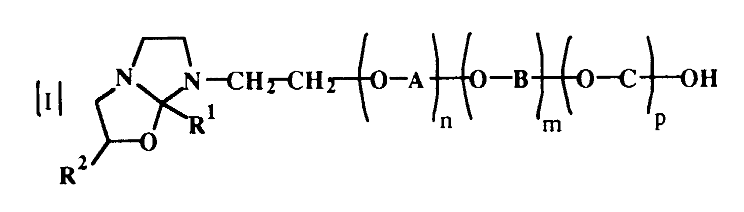 dans laquelle R¹ représente un atome d'hydrogène ou un groupement hydrocarboné ayant de 1 à 40 atomes de carbone, R² représente un atome d'hydrogène ou un groupement hydrocarboné ayant de 1 à 12 atomes de carbone, A, B et C, identiques ou différents, représentent chacun un groupe hydrocarboné divalent ayant de 2 à 6 atomes de carbone, n est un nombre entier de 5 à 50, m et p, identiques ou différents, sont chacun zéro ou un nombre entier de 1 à 25, et la somme n+m+p est un nombre entier de 5 à 50, le dit constituant (L) consistant en au moins un composé choisi dans le groupe formé par les produits détergents-dispersants, et le dit constituant (M) consistant en au moins un composé choisi dans le groupe formé par les huiles lubrifiantes minérales ou synthétiques et les polyglycols solubles dans ledit carburant.

2. Formulation selon la revendication 1 dans laquelle le constituant (K) est choisi parmi les composés de formule générale (I) dans laquelle R¹ représente un atome d'hydrogène ou un groupement hydrocarboné ayant de 4 à 25 atomes de carbone, R² représente un atome d'hydrogène ou un groupement hydrocarboné ayant de 1 à 6 atomes de carbone, A, B et C, identiques ou différents, représentent chacun un groupe hydrocarboné divalent ayant de 2 à 4 atomes de carbone, n est un nombre entier de 10 à 50, m et p, identiques ou différents, sont chacun zéro ou un nombre entier de 1 à 25, et la somme n+m+p est un nombre entier de 10 à 50.

3. Formulation selon la revendication 1 ou 2 dans laquelle le constituant (K) est choisi parmi les composés de formule générale (I) dans laquelle R¹ représente un atome d'hydrogène ou un groupement hydrocarboné ayant de 4 à 25 atomes de carbone, R² représente un atome d'hydrogène ou un groupement hydrocarboné ayant de 1 à 3 atomes de carbone, A, B et C, identiques ou différents, représentent chacun un groupe hydrocarboné divalent ayant de 2 à 4 atomes de carbone, n est un nombre entier de 10 à 25, m et p, identiques ou différents, sont chacun zéro ou un nombre entier de 1 à 25, et la somme n+m+p est un nombre entier de 10 à 25.

4. Formulation selon l'une des revendications 1 à 3 dans laquelle le constituant (K) est choisi parmi les composés de formule générale (I) dans laquelle R¹ représente un atome d'hydrogène ou un groupement alcoyle, alcényle, aryle, alkaryle ou aralkyle, R² représente un atome d'hydrogène ou groupement alcoyle, linéaire ou ramifié et de préférence linéaire, m et p sont égaux à zéro et A représente le groupe diméthylène, le groupe méthyl-1 diméthylène ou le groupe éthyle-1 diméthylène.

5. Formulation selon l'une des revendications 1 à 4 dans laquelle le constituant (K) est choisi parmi les composés de formule générale (I) dans laquelle R¹ représente un atome d'hydrogène ou un groupement alcoyle ou alcényle, linéaire ou ramifié.

6. Formulation selon l'une des revendications 1 à 5 dans laquelle le constituant (L) est choisi dans le groupe formé par les polyoléfines, les polyisobutène-amines, les mélanges de ces types de composés, les produits résultant de la réaction dans une première étape d'au moins un dérivé succinique choisi dans le groupe formé par les acides et les anhydrides alcénylsucciniques et les acides et les anhydrides polyalcénylsucciniques sur au moins une 1-(2-hydroxy-éthyl-)imidazoline substituée en position 2 par un radical alkyle ou alcényle, linéaire ou ramifié, ayant de 1 à 25 atomes de carbone, le rapport molaire imidazoline/dérivé succinique étant de 0,1 : 1 à 0,9 : 1, ladite étape étant effectuée dans des conditions telles que l'on forme et que l'on élimine au moins 0,15 mole d'eau par mole d'imidazoline engagée ; et dans une deuxième étape de la réaction du produit issu de la première étape sur au moins une polyamine répondant à l'une des formules générales suivantes : 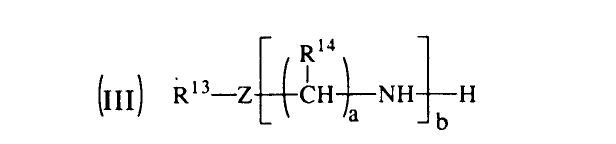 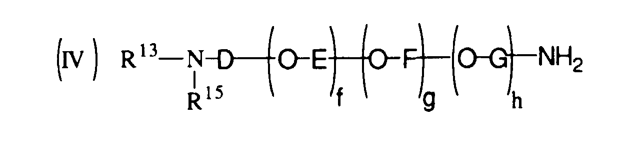 dans lesquelles R¹³ représente un atome d'hydrogène ou un groupe hydrocarboné ayant de 1 à 60 atomes de carbone, Z est choisi parmi les groupes -O-, et -NR¹⁵- dans lesquels R¹⁵ représente un atome d'hydrogène ou groupe hydrocarboné ayant de 1 à 60 atomes de carbone, R¹³ et R¹⁵ pouvant former ensemble avec l'atome d'azote auquel ils sont liés un hétérocycle, chacun des R¹⁴ indépendamment représente un atome d'hydrogène ou un groupe hydrocarboné ayant de 1 à 4 atomes de carbone, a est un nombre entier de 2 à 6, b est un nombre entier de 1 à 10 lorsque Z est -NR¹⁵- et un nombre entier de 2 à 10 lorsque Z est -O-, D, E, F et G, identiques ou différents, représentent chacun un groupe hydrocarboné divalent ayant de 2 à 6 atomes de carbone, f est un nombre entier de 1 à 60, g et h, identiques ou différents, sont chacun zéro ou un nombre entier de 1 à 50 et la somme f + g + h est un nombre entier de 1 à 60, la quantité de polyamine mise en réaction étant d'au moins 0,1 mole par mole de dérivé succinique introduit dans la première étape.

7. Formulation selon l'une des revendications 1 à 6 dans laquelle le constituant (L) est choisi dans le groupe formé par les produits résultant de la réaction dans une première étape d'au moins un dérivé succinique choisi dans le groupe formé par les anhydrides alcénylsucciniques ou polyalcénylsucciniques de masse moléculaire moyenne en nombre de 200 à 3 000 sur au moins une 1-(2-hydroxy-éthyl)imidazoline substituée en position 2 choisie dans le groupe formé par la 1-(2-hydroxyéthyl)-2-heptadécénylimidazoline et la 1-(2-hydroxyéthyl)-2-méthylimidazoline ; et dans une deuxième étape de la réaction du produit issu de la première étape sur au moins une polyamine répondant à l'une des formules générales suivantes : 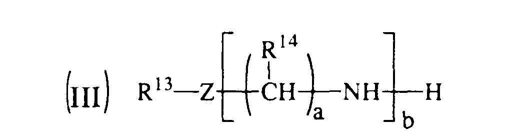 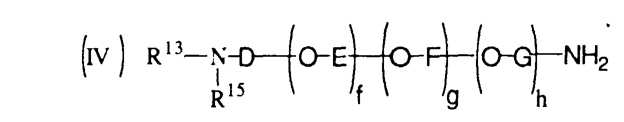 dans lesquelles Z représente un groupe -NR¹⁵-, R¹³, R¹⁴ et R¹⁵ représentent chacun un atome d'hydrogène, a est égal à 2 et b est un nombre entier de 1 à 5, D, E ,F et G, identiques ou différents représentent chacun un groupe alkylène ayant de 2 à 4 atomes de carbone, f est un nombre entier de 1 à 60 et g et h sont égaux à zéro ou f est un nombre entier de 1 à 59, h est zéro ou un nombre entier tel que la somme f + h soit de 1 à 59 et g est un nombre entier de 1 à 50, avec dans chaque cas la somme f + g + h égale à un nombre entier de 1 à 60.

8. Formulation selon l'une des revendications 1 à 7 dans laquelle le constituant (L) est choisi dans le groupe formé par les polyisobutènes, les polyisobutène-amines, les mélanges de ces types de composés et de préférence par les mélanges contenant une proportion minoritaire de polyisobutènes et une proportion majoritaire de polyisobtutène-éthylène-diamines.

9. Formulation selon l'une des revendications 1 à 8 dans laquelle le constituant (M) est choisi dans le groupe formé par les huiles lubrifiantes minérales ou synthétiques et les polyglycols, solubles dans ledit carburant, ayant une masse moléculaire moyenne en nombre de 480 à 2 100 et de formule générale (VIII) : 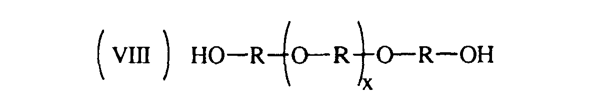 dans laquelle chacun des groupes R indépendamment représente un groupe hydrocarboné ayant de 2 à 6 atomes de carbone et x représente le degré moyen de polymérisation.

10. Formulation selon l'une des revendications 1 à 9 dans laquelle le constituant (M) est un polyglycol, ayant un indice de polydispersité de 1 à 1,25, de formule générale (VIII) dans laquelle chacun des groupes R indépendamment représente un groupe alkylène, linéaire ou ramifié, ayant de 2 à 4 atomes de carbone de préférence un groupe éthylène ou propylène.

11. Formulation selon l'une des revendications 1 à 10 dans laquelle le constituant (M) est un polyglycol, de formule générale (VIII) dans laquelle chacun des groupes R indépendamment représente un groupe propylène de formule : 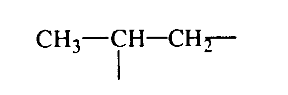 et a une masse moléculaire moyenne en nombre de 600 à 1 800 et de préférence de 650 à 1 250.

12. Utilisation d'une formulation d'additifs selon l'une des revendications 1 à 11 comme additif pour carburant à base d'hydrocarbures ou d'un mélange d'hydrocarbures et d'au moins un composé oxygéné choisi dans le groupe formé par les alcools et les éthers.

13. Utilisation d'une formulation d'additifs selon l'une des revendications 1 à 11 comme additif pour un carburant employé dans les moteurs à allumage commandé.

14. Utilisation selon la revendication 12 ou 13 dans laquelle ledit carburant contient de 10 à 10 000 ppm en poids de ladite formulation d'additifs.

15. Utilisation selon la revendication 14 dans laquelle la formulation comprend les constituants (K), (L) et (M) dans un rapport pondéral [ (K)/(L) ] de 0,02 : 1 à 4:1 et [ (L)/(M) ] de 0.05:1 à 10:1.

16. Composé hétérocyclique de type imidazo-oxazole, comportant une chaîne latérale alkoxylée, de formule générale (I) : 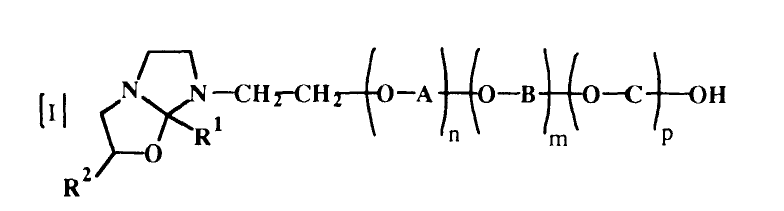 dans laquelle R¹ représente un atome d'hydrogène ou un groupement hydrocarboné ayant de 1 à 40 atomes de carbone, R² représente un atome d'hydrogène ou un groupement hydrocarboné ayant de 1 à 12 atomes de carbone, A, B et C, identiques ou différents, représentent chacun un groupe hydrocarboné divalent ayant de 2 à 6 atomes de carbone, n est un nombre entier de 5 à 50, m et p, identiques ou différents, sont chacun zéro ou un nombre entier de 1 à 25, et la somme n+m+p est un nombre entier de 5 à 50.

17. Composé selon la revendication 16 de formule générale (I) dans laquelle R¹ représente un atome d'hydrogène ou un groupement hydrocarboné ayant de 4 à 25 atomes de carbone, R² représente un atome d'hydrogène ou un groupement hydrocarboné ayant de 1 à 6 atomes de carbone, A, B et C, identiques ou différents, représentent chacun un groupe hydrocarboné divalent ayant de 2 à 4 atomes de carbone, n est un nombre entier de 10 à 50, m et p, identiques ou différents, sont chacun zéro ou un nombre entier de 1 à 25, et la somme n+m+p est un nombre entier de 10 à 50.

18. Composé selon la revendication 16 ou 17 de formule générale (I) dans laquelle R¹ représente un atome d'hydrogène ou un groupement hydrocarboné ayant de 4 à 25 atomes de carbone, R² représente un atome d'hydrogène ou un groupement hydrocarboné ayant de 1 à 3 atomes de carbone, A, B et C, identiques ou différents, représentent chacun un groupe hydrocarboné divalent ayant de 2 à 4 atomes de carbone, n est un nombre entier de 10 à 25, m et p, identiques ou différents, sont chacun zéro ou un nombre entier de 1 à 25, et la somme n+m+p est un nombre entier de 10 à 25.

19. Composé selon l'une des revendications 16 à 18 de formule générale (I) dans laquelle R¹ représente un atome d'hydrogène ou un groupement alcoyle, alcényle, aryle, alkaryle ou aralkyle, R² représente un atome d'hydrogène ou groupement alcoyle, linéaire ou ramifié et de préférence linéaire, m et p sont égaux à zéro et A représente le groupe diméthylène, le groupe méthyl-1 diméthylène ou le groupe éthyle-1 diméthylène.

20. Composé selon l'une des revendications 16 à 19 de formule générale (I) dans laquelle R¹ représente un atome d'hydrogène ou un groupement alcoyle ou alcényle, linéaire ou ramifié.

## Patentansprüche

1. Zusätzeformulierung, insbesondere für Treibstoffe, dadurch gekennzeichnet, daß sie mindestens eine Komponente (K), mindestens eine Komponente (L) und mindestens eine Komponente (M) beinhalter, wobei die besagte Komponente (K) aus mindestens einer heterocyclischen Verbindung imidazooxazolischer Art besteht, eine alkoxylierte Seitenkette enthält, gemäß der Summenformel (1) : 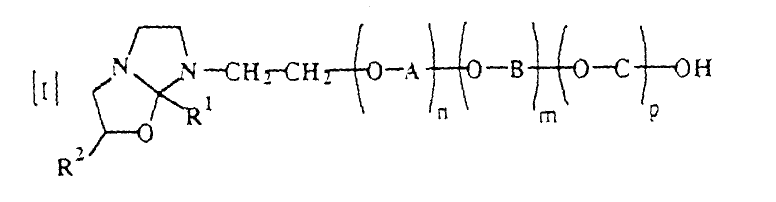 in der R¹ ein Wasserstoffatom oder eine teerhaltige Gruppierung mit 1 bis 40 Kohlenstoffatomen, R² ein Wasserstoffatom oder eine teerhaltige Gruppierung mit 1 bis 12 Kohlenstoffatomen darstellt, wobei A, B, und C identisch oder unterschiedlich sind und jeweils eine zweiwertige teerhaltige Gruppe mit 2 bis 6 Kohlenstoffatomen darstellen, n ist eine ganze Zahl zwischen 5 und 50, m und p - ob identisch oder unterschiedlich - sind je gleich Null oder eine ganze Zahl von 1 bis 25, die Summe n+m+p ist eine ganze Zahl von 5 bis 50, wobei die besagte Komponente (L) aus mindestens einer Verbindung besteht, die in der Gruppe der Detergens-Dispergiermittel gewählt wurde, und die besagte Komponente (M) aus mindestens einer Verbindung besteht, die in der Gruppe der mineralischen oder synthetischen Schmieröle und der in dem besagten Treibstoff löslischen Polyglykole ausgesucht wurde.

2. Formulierung gemäß Anspruch 1, in der die Komponente (K) unter den Verbindungen nach der Summenformel (I) ausgesucht wird, in der R¹ ein Wasserstoffatom oder eine teerhaltige Gruppierung mit 4 bis 25 Kohlenstoffatomen, R² ein Wasserstoffatom oder eine teerhaltige Gruppierung mit 1 bis 6 Kohlenstoffatomen darstellt, wobei A, B, und C identisch oder unterschiedlich sind und jeweils eine zweiwertige teerhaltige Gruppe mit 2 bis 4 Kohlenstoffatomen darstellen, n ist eine ganze Zahl zwischen 10 und 50, m und p - ob identisch oder unterschiedlich - sind je gleich Null oder eine ganze Zahl von 1 bis 25, die Summe n+m+p ist eine ganze Zahl von 10 bis 50.

3. Formulierung gemäß Anspruch 1 oder 2, in der die Komponente (K) unter den Verbindungen nach der Summenformel (I) ausgesucht wird, in der R¹ ein Wasserstoffatom oder eine teerhaltige Gruppierung mit 4 bis 25 Kohlenstoffatom, R² ein Wasserstoffatom oder eine teerhaltige Gruppierung mit 1 bis 31 Kohlenstoffatomen darstellt, wobei A, B, und C identisch oder unterschiedlich sind und jeweils eine zweiwertige teerhaltige Gruppe mit 2 bis 4 Kohlenstoffatomen darstellen, n ist eine ganze Zahl zwischen 10 und 25, m und p - ob identisch oder unterschiedlich - sind je gleich Null oder eine ganze Zahl von 1 bis 25, die Summe n+m+p ist eine ganze Zahl von 10 bis 25.

4. Formulierung gemäß einem der Ansprüche 1 bis 3, in der die Komponente (K) unter den Verbindungen nach der Summenformel (I) ausgesucht wird, in der R¹ ein Wasserstoffatom oder eine Alkyl-, Alkenyl-, Aryl-, Alkaryl- oder Aralkylgruppierung, R² ein Wasserstoffatom oder eine Alkylgruppierung, die geradkettig oder verzweigt, vorzugsweise geradkettig, ist, m und p sind gleich Null und A stellt die Dimethylengruppe, die Methyl-1 dimethylen-Gruppe oder die Ethyl-1 dimethylen-Gruppe dar.

5. Formulierung gemäß einem der Ansprüche 1 bis 4, in der die Komponente (K) unter den Verbindung nach der Summenformel (I) ausgesucht wird, in der R¹ ein Wasserstoffatom oder eine Alkyl-, Alkenylgruppierung, die geradkettig oder verzweigt ist, darstellt.

6. Formulierung gemäß einem der Ansprüche 1 bis 5, in der die Komponente (L), in der Gruppe der Polyolefine, der Polyisobutenamine, der Gemische aus diesen Verbindungsarten, der Produkte aus der Reaktion in einer ersten Stufe von mindestens einem Bernsteinsäurederivat aus der Gruppe der Alkenylbernsteinsäuren und -anhydride und der Polyalkenyl-bernsteinsäuren und -anhydride mit mindestens einem 1-(2-Hydroxy-Ethyl)imidazolin, das in der Position 2 durch ein geradkettiges oder ein verzweigtes Alkyl- oder Alkenylradikal mit 1 bis 25 Kohlenstoffatomen ersetzt wird, ausgesucht wird, wobei das Molverhältnis zwischen dem Imidazolin und dem Bernsteinsäurederivat 0,1 : 1 bis 0,9 : 1 beträgt; die besagte Stufe wird unter solchen Bedingungen durchgeführt, daß mindestens 0,15 Mol Wasser pro Mol des eingesetzten Imidazolins entsteht und entzogen wird; und in einer zweiten Stufe aus der Reaktion des aus der ersten Stufe stammenden Produktes mit mindestens einem Polyamin, das einer der folgenden Summenformeln entspricht: 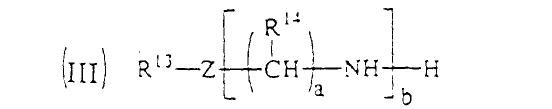 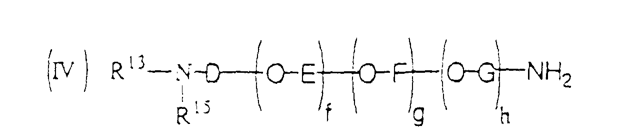 in denen R¹³ ein Wasserstoffatom oder eine teerhaltige Gruppe mit1 bis 60 Kohlenstoffatomen darstellt, Z unter den -O- und -NR¹⁵- Gruppen ausgesucht wird, in denen R¹⁵ ein Wasserstoffatom oder eine teerhaltige Gruppe mit 1 bis 60 Kohlenstoffatomen ist, wobei R¹³ und R¹⁵ zusammen mit dem Stickstoffatom, an dem sie gebunden sind, eine heterocyclische Verbindung bilden können; jeder einzelne R¹⁴ stellt ein Wasserstoffatom oder eine teerhaltige Gruppe mit 1 bis 4 Kohlenstoffatomen dar, a ist eine ganze Zahl von 2 bis 6, b eine ganze Zahl von 1 bis 10, wenn Z eine -NR¹⁵-Gruppe bedeutet, eine ganze Zahl von 2 bis 10, wenn Z -O-, D, E, F, und G -ob identisch oder unterschiedlich- je eine zweiwertige teerhaltige Gruppe mit 2 bis 6 Kohlenstoffatomen darstellen, f ist eine ganze Zahl von 1 bis 60, g und h - ob identisch oder unterschiedlich - bedeuten je Null oder eine ganze Zahl von 1 bis 50, und die Summe f + g + h ist eine ganze Zahl von 1 bis 60, wobei die für die Reaktion verwendete Polyaminmenge mindestens 0,1 Mol pro Mol des in die erste Phase eingebrachten Bernsteinsäurederivats beträgt.

7. Formulierung gemäß einem der Ansprüche 1 bis 6, in der die Komponente (L) in der Gruppe der Produkteausgesucht wird, die aus der Reaktion in einer ersten Stufe von mindestens einem Bernsteinsäurederivat aus der Gruppe der Alkenylbernsteinsäureanhydride oder der Polyalkenylbernsteinsaureanhydride mit einer durchschnittlichen Molmasse von 200 bis 3 000 mit mindestens einem 1-(2-Hydroxyethyl-)imidazolin hervorgehen, das in der Position 2 der Gruppe des 1-(2-Hydroxethyl) -2-heptadecenylimdazolin und des 1-(2-Hydroxyethyl)-2-methylimidazolin substituiert wird; und in einer zweiten Stufe aus der Reaktion des aus der ersten Stufe stammenden Produktes mit mindestens einem Polyamin, das einer der folgenden Summenformeln entspricht: 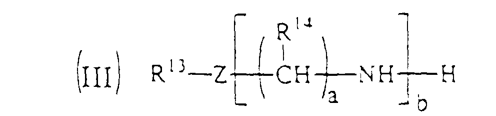 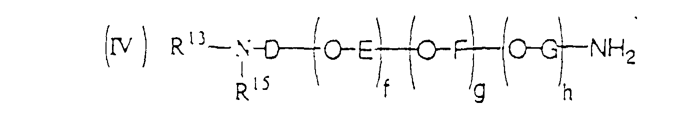 in denen Z eine NR¹⁵-Gruppe darstellt, R¹³, R¹⁴, R¹⁵ je ein Wasserstoffatom sind, a ist gleich 2 und b ist eine ganze Zahl von 1 bis 5, D, E, F, und G - ob identisch oder unterschiedlich - je eine Alkylengruppe mit 2 bis 4 Kohlenstoffatomen darstellen, f ist eine ganze Zahl von 1 bis 60, g und h bedeuten je Null oder f ist eine ganze Zahl von 1 bis 59, h ist gleich Null oder eine ganze Zahl, sodass die Summe f + h 1 bis 59 beträgt, und g ist eine ganze Zahl von 1 bis 50, wobei die Summe f + g + h in jedem Fall eine ganze Zahl von 1 bis 60 ist.

8. Formulierung gemäß einem der Ansprüche 1 bis 7, in der die Komponente (L) in der Gruppe der Polyisobutene, der Polyisobutenamine, der Gemische aus diesen Verbindungsarten ausgesucht wird, vorzugsweise in Gemischen, die einen Minderanteil an Polyisobutenen und einen Hauptanteil an Polyisobuten-Ethylen-Diaminen enthalten.

9. Formulierung gemäß einem der Ansprüche 1 bis 8, in der die Komponente (M) in der Gruppe der mineralischen oder synthetischen Schmieröle und der Polyglykole, die in dem besagten Treibstoff löslich sind und eine durchschnittliche Molmasse von 480 bis 2 100 aufweisen, ausgesucht wird, und folgende Summenformel hat (VII) : 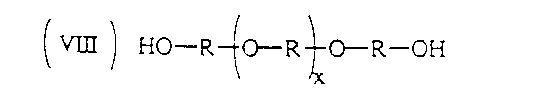 in der jede einzelne R-Gruppe eine teerhaltige Gruppe mit 2 bis 6 Kohlenstoffatomen darstellt und x der durchschnittliche Polymerisationsgrad ist.

10. Formulierung gemäß einem der Ansprüche 1 bis 9, in der die Komponente (M) ein Polyglykol mit einem Polydispersitätsgrad von 1 bis 1,25 mit der Summenformel (VIII) ist, in der jede einzelne R-Gruppe eine geradkettige oder verzweigte Alkylengruppe mit 2 bis 4 Kohlenstoffatomen, vorzugsweise eine Ethylen- oder eine Propylengruppe, darstellt.

11. Formulierung gemäß einem der Ansprüche 1 bis 10, in der die Komponente (M) ein Polyglykol mit der Summenformel (VIII) ist, in der jede einzelne R-Gruppe eine Propylengruppe folgender Formel darstellt : 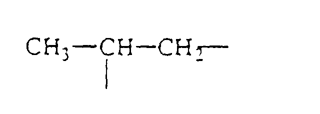 und a eine durchschnittliche Molmasse von 600 bis 1 800, vorzugsweise von 650 bis 1 250, aufweist.

12. Verwendung einer Zusätzeformulierung nach einem der Ansprüche 1 bis 11 als Zusatz für einen Treibsoff, der auf Kohlenwasserstoffen oder auf einer Mischung von Kohlenwasser-stoffen und mindestens einer Sauerstoffverbindung basiert, die in der Alkohol- und Ethergruppe gewählt wird.

13. Verwendung einer Zusätzeformulierung nach einem der Ansprüche 1 bis 11 als Zusatz für einen Treibstoff, der in Motoren mit gesteuerter Zündung verwenden wird.

14. Verwendung nach Anspruch 12 oder 13, bei der der besagte Treibstoff von 10 bis 10 000 ppm der Zusätzeformulierung im Gewicht enthält.

15. Verwendung nach Anspruch 14, bei der die Formulierung die Komponente (K), (L) und (M) in einem Gewichtsverhältnis [(K)/(L)] von 0,02 : 1 bis 4 : 1 und [(L)/(M)] in einem Gewichtsverhältnis von 0,05 : 1 bis 10 : 1 enthält.

16. Heterocyclische Verbindung imidazo-oxazolischer Art, die eine alkoxylierte Seitenkette enthält und folgende Summenformel aufweist (I) : 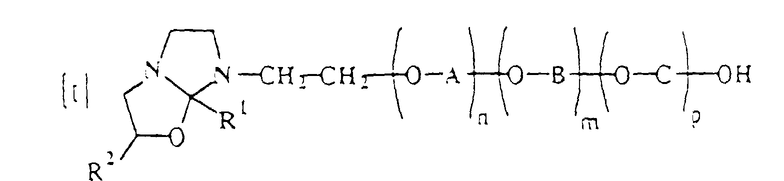 in der R¹ ein Wasserstoffatom oder eine teerhaltige Gruppierung mit 1 bis 40 Kohlenstoffatomen, R² ein Wasserstoffatom oder eine teerhaltige Gruppierung mit 1 bis 12 Kohlenstoffatomen darstellt, wobei A, B, und C identisch oder unterschiedlich sind und jeweils eine zweiwertige teerhaltige Gruppe mit 2 bis 6 Kohlenstoffatomen darstellen, n ist eine ganze Zahl von 5 bis 50, m und p - ob identisch oder unterschiedlich - sind je gleich Null oder eine ganze Zahl von 1 bis 25, die Summe n + m + p ist eine ganze Zahl von 5 bis 50.

17. Verbindung gemäß Anspruch 16 nach der Summenformel (I), in der R¹ ein Wasserstoffatom oder eine teerhaltige Gruppierung mit 4 bis 25 Kohlenstoffatomen, R² ein Wasserstoffatom oder eine teerhaltige Gruppierung mit 1 bis 6 Kohlenstoffatomen darstellt, wobei A, B, und C identisch oder unterschiedlich sind und jeweils eine zweiwertige teerhaltige Gruppe mit 2 bis 4 Kohlenstoffatomen darstellen, n ist eine ganze Zahl zwischen 10 und 50, m und p - ob identisch oder unterschiedlich - sind je gleich Null oder eine ganze Zahl von 1 bis 25, die Summe n + m + p ist eine ganze Zahl von 10 bis 50.

18. Verbindung gemäß Anspruch 16 oder 17 nach der Summenformel (I), in der R¹ ein Wasserstoffatom oder eine teerhaltige Gruppierung mit 4 bis 25 Kohlenstoffatomen, R² ein Wasserstoffatom oder eine teerhaltige Gruppierung mit 1 bis 3 Kohlenstoffatomen darstellt, wobei A, B, und C identisch oder unterschiedlich sind und jeweils eine zweiwertige teerhaltige Gruppe mit 2 bis 4 Kohlenstoffatomen darstellen, n ist eine ganze Zahl zwischen 10 und 25, m und p - ob identisch oder unterschiedlich - sind je gleich Null oder eine ganze Zahl von 1 bis 25, die Summe n + m + p ist eine ganze Zahl von 10 bis 25.

19. Verbindung gemäß einem der Ansprüche 16 bis 18 nach der Summenformel (I), in der R¹ ein Wasserstoffatom oder eine Alkyl-, Alkenyl-, Aryl-, Alkaryl- oder Aralkylgruppierung, R² ein Wasserstoffatom oder eine Alkylgruppierung, die geradkettig oder verzweigt, vorzugsweise geradkettig, ist, m und p sind gleich Null und A stellt die Dimethylengruppe, die Methyl-1 dimethylen-Gruppe oder die Ethyl-1 dimethylen-Gruppe dar.

20. Verbindung gemäß einem der Ansprüche 16 bis 19 nach der Summenformel (I), in der R¹ ein Wasserstoffatom oder eine Alkyl- oder eine Alkenylgruppierung, die geradkettig oder verzweigt ist, darstellt.

## Claims

1. Additive formulation, particularly for a fuel, characterised in that it comprises at least one constituent (K), at least one constituent (L) and at least one constituent (M), said constituent (K) consisting of at least one heterocyclic imidazo-oxazole type compound containing an alkoxylated side chain and having general formula (I): 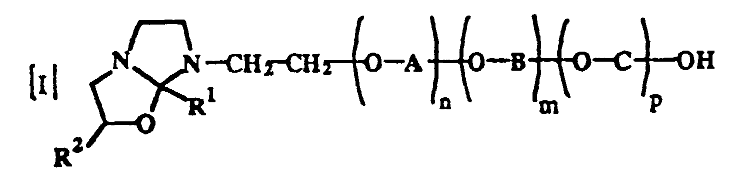 where R¹ represents a hydrogen atom or a hydrocarbon group containing 1 to 40 carbon atoms, R² represents a hydrogen atom or a hydrocarbon group containing 1 to 12 carbon atoms, A, B, and C, which may be identical or different, each represent a divalent hydrocarbon group containing 2 to 6 carbon atoms, n is a whole number from 5 to 50, m and p, which may be identical or different, are each zero or a whole number from 1 to 25, and the sum n+m+p is a whole number from 6 to 50, said constituent (L) consisting of at least one compound selected from the group formed by detergent-dispersant products, and said constituent (M) consisting of at least one compound selected from the group formed by mineral or synthetic lubricating oils and polyglycols which are soluble in said fuel.

2. Formulation according to claim 1 where constituent (K) is selected from compounds with general formula (I) where R¹ represents a hydrogen atom or a hydrocarbon group containing 4 to 25 carbon atoms, R² represents a hydrogen atom or a hydrocarbon group containing 1 to 6 carbon atoms, A, B and C, which may be identical or different, each represent a divalent hydrocarbon group containing 2 to 4 carbon atoms, n is a whole number from 10 to 50 , m and p, which may be identical or different, are each zero or a whole number from 1 to 25, and the sum n+m+p is a whole number from 10 to 50.

3. Formulation according to claim 1 or claim 2 wherein constituent (K) is selected from compounds with general formula (I) where R¹ represents a hydrogen atom or a hydrocarbon group containing 4 to 25 carbon atoms, R² represents a hydrogen atom or a hydrocarbon group containing 1 to 3 carbon atoms, A, B and C, which may be identical or different, each represent a divalent hydrocarbon group containing 2 to 4 carbon atoms, n is a whole number from 10 to 25, m and p, which may be identical or different, are each zero or a whole number from 1 to 25, and the sum n+m+p is a whole number from 10 to 25.

4. Formulation according to any one of claims 1 to 3 wherein constituent (K) is selected from compounds with general formula (I) where R¹ represents a hydrogen atom or an alkoyl, alkenyl, aryl, alkaryl or aralkyl group, R² represents a hydrogen atom or a linear or branched, preferably linear, alkoyl group, m and p equal zero and A represents a dimethylene, 1-methyl dimethylene or 1-ethyl dimethylene group.

5. Formulation according to any one of claims 1 to 4 wherein constituent (K) is selected from compounds with general formula (I) where R¹ represents a hydrogen atom or a linear or branched alkoyl or alkenyl group.

6. Formulation according to any one of claims 1 to 5 wherein constituent (L) is selected from the group formed by polyolefins, polyisobutene-amines, or mixtures of these compounds, the products resulting from reaction in a first step of at least one succinic derivative selected from the group formed by alkenylsuccinic acids and anhydrides and polyalkenylsuccinic acids and anhydrides with at least one 1-(2-hydroxyethyl)-imidazoline substituted in the 2 position by a linear or branched alkyl or alkenyl radical containing 1 to 25 carbon atoms, the imidazoline/succinic derivative molar ratio being from 0.1:1 to 0.9:1, said step being carried out under conditions such that at least 0.15 moles of water per mole of imidazoline used is formed and eliminated; and in a second step of the reaction, the product from the first step is reacted with at least one polyamine having one of the following general formulae: 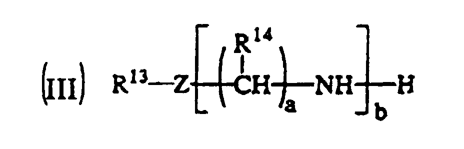 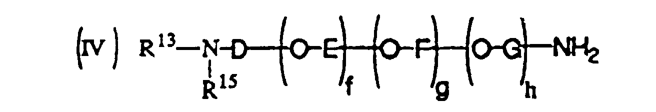 where R¹³ represents a hydrogen atom or a hydrocarbon group containing 1 to 60 carbon atoms, Z is selected from the groups -O- and -NR¹⁵-, where R¹⁵ represents a hydrogen atom or a hydrocarbon group containing 1 to 60 carbon atoms, R¹³ and R¹⁵ being capable of forming a heterocycle with the nitrogen atom to which they are bonded, each R¹⁴ group independently represents a hydrogen atom or a hydrocarbon group containing 1 to 4 carbon atoms, a is a whole number from 2 to 6, b is a whole number from 1 to 10 when Z is -NR¹⁵- and a whole number from 2 to 10 when Z is -O-, D, E, F and G, which may be identical or different, each represent a divalent hydrocarbon group containing 2 to 6 carbon atoms, f is a whole number from 1 to 60, g and h, which may be identical or different, are each zero or a whole number from 1 to 50 and the sum f+g+h is a whole number from 1 to 60, the quantity of polyamine used in the reaction being at least 0.1 mole per mole of succinic derivative introduced in the first step.

7. Formulation according to any one of claims 1 to 6 wherein constituent (L) is selected from the group formed by the products resulting from reaction in a first step with at least one succinic derivative selected from the group formed by alkenylsuccinic or polyalkenylsuccinic anhydrides with an average molecular weight of 200 to 3000 with at least one 1-(2-hydroxyethyl)-imidazoline substituted in the 2 position selected from the group formed by 1-(2-hydroxyethyl)-2-heptadecenylimidazoline and 1-(2-hydroxyethyl)-2-methylimidazoline; and in a second step of the reaction, the product from the first step is reacted with at least one polyamine having one of the following general formulae: 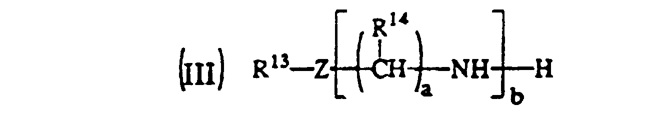 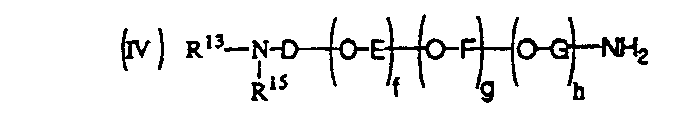 where Z represents a -NR¹⁵- group, R¹³, R¹⁴ and R¹⁵ each represent a hydrogen atom, a equals 2 and b is a whole number from 1 to 5, D, E, F and G, which may be identical or different, each represent an alkylene group containing 2 to 4 carbon atoms, f is a whole number from i to 60 and g and h equal zero or f is a whole number from 1 to 59, h is zero or a whole number such that the sum f+h is from 1 to 59 and g is a whole number from 1 to 50, and in each case the sum f+g+h is a whole number from 1 to 60.

8. Formulation according to any one of claims 1 to 7 wherein constituent (L) is selected from the group formed by polyisobutenes, polyisobutene-amines and mixtures of these compounds, preferably by mixtures containing a minor proportion of polyisobutenes and a major proportion of polyisobutene-ethylenediamines.

9. Formulation according to any one of claims 1 to 8 wherein constituent (M) selected from the group formed by mineral or synthetic lubricating oils and polyglycols which are soluble in said fuel, preferably with an average molecular weight of 480 to 2100 and general formula (VIII): 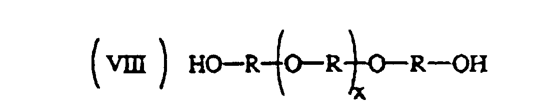 where each group R independently represents a hydrocarbon group containing 2 to 6 carbon atoms and x represents the average degree of polymerisation.

10. Formulation according to any one of claims 1 to 9 wherein constituent (M) is a polyglycol having a polydispersity index of 1 to 1.25, with general formula (VIII) where each group R independently represents a linear or branched alkylene group containing 2 to 4 carbon atoms, preferably an ethylene or propylene group.

11. Formulation according to any one of claims 1 to 10 wherein constituent (M) is a polyglycol with general formula (VIII) where each group R independently represents a propylene group with formula: 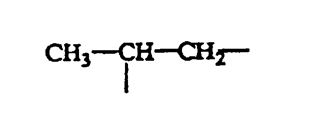 with an average molecular weight of 600 to 1800, preferably 650 to 1250.

12. Use of an additive formulation according to any one of claims 1 to 11 as an additive for a fuel based on a hydrocarbon or a mixture of hydrocarbons and at least one oxygenated compound selected from the group formed by alcohols and ethers.

13. Use of an additive formulation according to any one of claims 1 to 11 as an additive for a fuel used in spark ignition engines.

14. Use according to claim 12 or 13 wherein said fuel contains from 10 to 10,000 ppm by weight of said additive formulation.

15. Use according to claim 14 wherein the additive formulation comprises constituents (K), (L) and (M) in a weight ratio [(K) / (L)] of 0.02:1 to 4:1 and [(L) / (M)] of 0.05:1 to 10:1.

16. A heterocyclic imidazo-oxazole type compound containing an alkoxylated side chain and having general formula (I) : 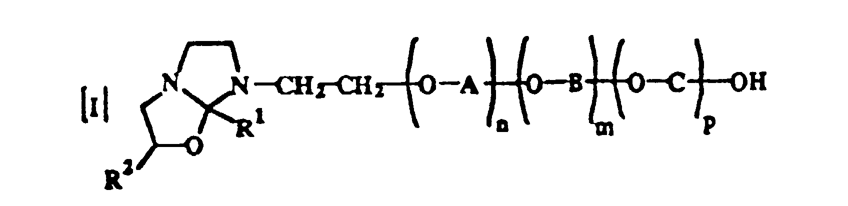 where R¹ represents a hydrogen atom or a hydrocarbon group containing 1 to 40 carbon atoms, R² represents a hydrogen atom or a hydrocarbon group containing 1 to 12 carbon atoms, A, B, and C, which may be identical or different, each represent a divalent hydrocarbon group containing 2 to 6 carbon atoms, n is a whole number from 5 to 50, m and p, which may be identical or different, are each zero or a whole number from 1 to 25, and the sum n+m+p is a whole number from 5 to 50.

17. A compound according to claim 16 with general formula (I) where R¹ represents a hydrogen atom or a hydrocarbon group containing 4 to 25 carbon atoms, R² represents a hydrogen atom or a hydrocarbon group containing 1 to 6 carbon atoms, A, B, and C, which may be identical or different, each represent a divalent hydrocarbon group containing 2 to 4 carbon atoms. n is a whole number from 10 to 50, m and p, which may be identical or different, are each zero or a whole number from 1 to 25, and the sum n+m+p is a whole number from 10 to 50.

18. A compound according to claim 16 or claim 17 with general formula (I) where R¹ represents a hydrogen atom or a hydrocarbon group containing 4 to 25 carbon atoms, R² represents a hydrogen atom or a hydrocarbon group containing 1 to 3 carbon atoms, A, B, and C, which may be identical or different, each represent a divalent hydrocarbon group containing 2 to 4 carbon atoms, n is a whole number from 10 to 25, m and p, which may be identical or different, are each zero or a whole number from 1 to 25, and the sum n+m+p is a whole number from 10 to 25.

19. A compound according to any one of claims 16 to 18 with general formula (I) where R¹ represents a hydrogen atom or an alkoyl, alkenyl, aryl, alkaryl or aralkyl group, R² represents a hydrogen atom or a linear or branched, preferably linear, alkoyl group, m and p equal zero and A represents a dimethylene, 1-methyl dimethylene or 1-ethyl dimethylene group.

20. A compound according to any one of claims 16 to 19 with general formula (I) where R¹ represents a hydrogen atom or a linear or branched alkoyl or alkenyl group.
